# EUROPEAN PATENT SPECIFICATION

(11) **EP 0 941 989 B1**
(45) Date of publication and mention of the grant of the patent: **08.07.2009**
(21) Application number: 99810156.2
(22) Date of filing: 22.02.1999
(51) Int. Cl.: C07D 251/24

(54) **Process for the preparation of 2,4-diaryl-6-o-hydroxyphenyl-1,3,5-triazine derivatives in the presence of a protic acid catalyst**
Verfahren zur Herstellung von 2,4-Diaryl-6-o-hydroxyphenyl-1,3,5-triazin Derivaten in Anwesenheit eines Protonensäure-Katalysators
Procédé de préparation de dérivés 2,4-diaryl-6-o-hydroxyphenyl-1,3,5-triazine en présence de catalyseur acide protique

(30) Priority: 02.03.1998 US 33266
(43) Date of publication of application: 15.09.1999
(73) Proprietor: Ciba Holding Inc., 4057 Basel (CH)
(72) Inventor: Stevenson, Tyler Arthur, Teaneck, NJ 07666 (US); Ackerman, Michael, Deceased (US); Hayoz, Pascal, 4114 Hofstetten (CH); Meuwly, Roger, 1784 Cournillens (CH); Oswald, John Francis, 5063 Wölflinswil (CH); Schregenberger, Christian, 4305 Olsberg (CH)

(56) References cited:
- EP-A- 0 165 608
- BE-A- 661 225
- FR-A- 1 466 836
- PATENT ABSTRACTS OF JAPAN vol. 97, no. 7, 31 July 1997 (1997-07-31) & JP 09 059263 A (ASAHI DENKA KOGYO K.K.), 4 March 1997 (1997-03-04)
- BRUNETTI H. & LÜTHI C.E.: "153. Die Synthese von asymmetrisch substituierten o-Hydroxyphenyl-s-triazinen" HELVETICA CHIMICA ACTA, vol. 55, no. 5, 1972, pages 1566-1595, XP002034121

## Description

This invention pertains to novel methods for the preparation of tris-aryl-o-hydroxyphenyl-s-triazines using cyanuric chloride as a starting material. Particular o-hydroxyphenyl-s-triazines which can be made by these new methods are: bis-aryl-resorcinol based s-triazines; mono-aryl-bis-resorcinol based s-triazines; and tris-resorcinol-s-triazines.

### Background of the Invention

Tris-aryl-o-hydroxyphenyl-s-triazines are intermediates for or are themselves UV absorbers useful for the protection of natural or synthetic materials from the adverse action of actinic radiation. There are many methods described for the preparation of such s-triazines as seen from the publications of S. Tanimoto and M. Yamagata, Senryo to Yakuhim, 1995, 40(12), 325-339 and of H. Brunetti and G. E. Luethi, Helv. Chim. Acta, 1972, 55(5), 1566-1595.

The most versatile method is to employ one or more Friedel-Crafts reactions starting from cyanuric chloride. A major obstacle in this approach is the fact that Friedel-Crafts reactions of aryl groups and cyanuric chloride are non-selective. This is a problem when the goal is to prepare an asymmetric tris-aryl-s-triazine. This is explained in the Tanimoto and Brunetti publications mentioned above and in United States Patent Nos. 5,084,570 and 5,106,972. However, it is well-known that substitution reactions between nucleophiles and cyanuric chloride are selective as taught by E. M. Smolin and L. Rapoport, s-Triazines and Derivatives in The Chemistry of Heterocyclic Compounds, A. Weissberger Ed., Interscience Publishers, New York, 1959, pp. 53-57.

One possible approach to prepare asymmetric tris-aryl-s-triazines selectively would be to (a) replace one or two chlorine atoms of cyanuric chloride with an appropriate protecting group in a nucleophilic manner; (b) replace the remaining chlorine atom(s) with the desired aryl group via a Friedel-Crafts reaction; (c) displace the protecting group(s) with chlorine, and finally (d) replace the newly formed chlorine(s) with the second aryl group in a Friedel-Crafts manner. United States Patent Nos. 5,084,570 and 5,106,972 disclose this strategy for the preparation of 2,(2,4-dihydroxyphenyl)-4,6-diaryl-s-triazine. The protecting group chosen in in these two patents is methyl mercaptan. The process outlined is four steps starting from cyanuric chloride.

Another drawback of Friedel-Crafts reactions of aryl groups and cyanuric chloride in addition of non-selectivity is the fact that large amounts of Lewis acid are necessary to mediate the reaction, normally equimolar amounts. The Lewis acid most commonly used is aluminum chloride. These reactions produce prodigious amounts of alumunim waste which is environmentally hard to handle. Some typical reactions using this catalyst are described in EP-A-165608.

Syntheses of some phenoxy substituted triazines are reported in BE-661225 and FR-1466836, Japanese Hei 9-59263 discloses a three-step synthetic approach for the preparation of asymmetric tris-aryl-hydroxyphenyl-s-triazines. The preferred method of the Japanese reference is a one-pot process using a Lewis acid to mediate all three steps. This reference will be discussed in more detail later in this application.

A welcome addition to the art therefore would be to (a) provide a method of performing Friedel-Crafts reactions between aryl groups and s-triazines using protic acids instead of Lewis acids, and to (b) provide a method of preparing asymmetric tris-aryl-s-triazines in less than four synthetic steps.

### Objects of the Invention

One object of the invention is a process in which protic acids may be employed as Friedel-Crafts catalysts for the reaction between aryl groups and s-triazines.

Another object of the invention is a method for the preparation of asymmetric tris-aryl-s-triazines in less than four synthetic steps.

A third object of the invention is a process combining the two processes mentioned above.

### Detailed Description of the Invention

Strong protic acids, such as hydrogen halides, sulfuric or sulfonic acids, nitric acid etc. as well as solid-supported protic acids such as AMBERLYST^{®} (Rohm & Haas),AMBERLITE^{®} (Rohm & Haas) and NAFION^{®} (duPont) catalysts, may be employed as effective Friedel-Crafts catalysts to form carbon-carbon bonds between aryl groups and s-triazines. Preferred are acids having a pKa value below 4, especially below 2; examples include the substances listed above or hydrogen chloride (gaseous or as solution), methanesulfonic acid, p-toluenesulfonic acid, phosphoric acid, polyphosphoric acid. Active leaving groups are halogen, especially Cl or F, or alkoxy and aryloxy. Preferred leaving groups are chlorine, phenoxy and substituted phenoxy moieties.

The simpliest example of a reaction of this type is replacing the chlorine atoms on cyanuric chloride with resorcinol to prepare a tris-2,4,6-(2,4-dihydroxyphenyl)-s-triazine. United States Patent Nos. 3,118,887 and 3.244.708 describe such syntheses, but where large amounts of aluminum chloride are used. Indeed, no added catalyst at all is required as the hydrogen chloride gas released during the initial nucleophilic reaction between resorcinol and cyanuric chloride serves as the catalyst for the carbon-carbon bond formation as seen below:

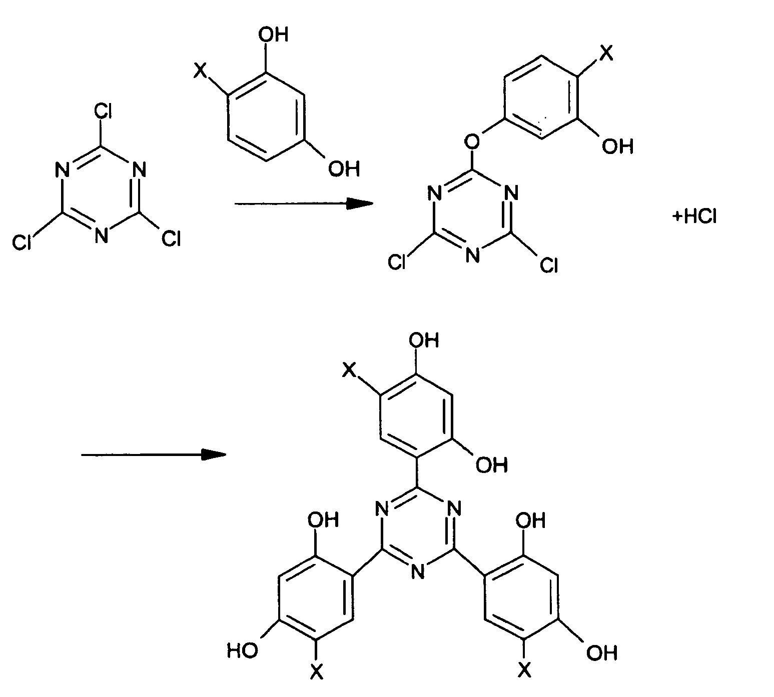

X in these formulae is hydrogen, alkyl, phenylalkyl or halogen. An effective temperature range is from 70°C to 200°C. The most effective temperature range is 100°C to 170°C.

This reaction may also be performed on mono-aryl-bis-chloro-s-triazines and bis-aryl-mono-chloro-s-triazines as seen below.

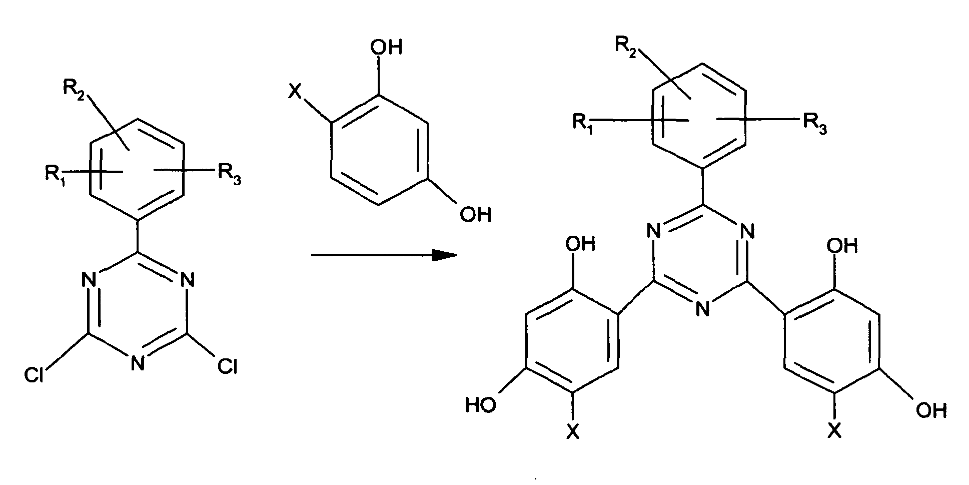

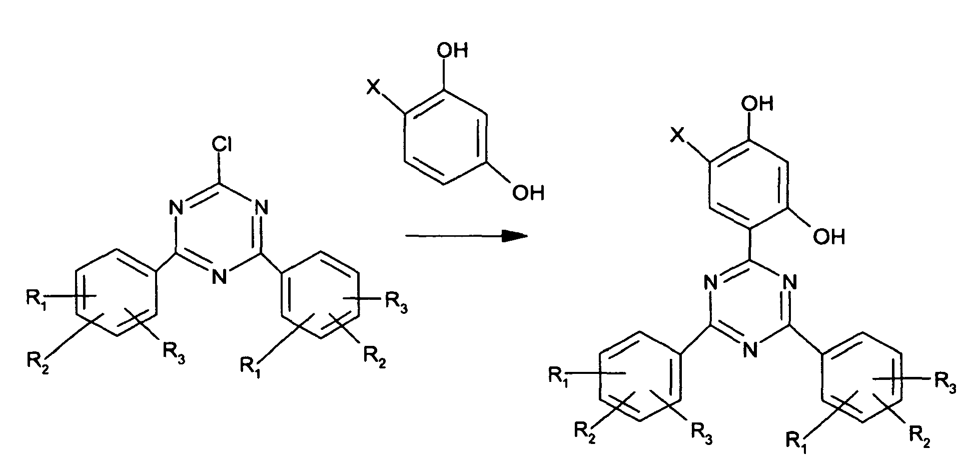

R₁, R₂ and R₃ are each independently hydrogen, alkyl of 1 to 12 carbon atoms, hydroxy, alkoxy of 1 to 12 carbon atoms, halogen, phenyl or phenyl substituted by alkyl of 1 to 12 carbon atoms, hydroxy, alkoxy of 1 to 12 carbon atoms or halogen. The solvents and temperature ranges are those given supra. O-Attached resorcinol groups on the s-triazine ring are observed as transient intermediates during the course of these reactions.

Similar Friedel-Crafts reactions may also be employed with alkoxy or aryloxy moieties as the leaving group. In these cases an external protic acid is added to the mixture. The most effective protic acids are hydrogen chloride gas and methanesulfonic acid. As with halogen, one or more alkoxy or aryloxy groups may be replaced in this fashion. Phenoxy and/or substituted phenoxy groups are especially suitable leaving groups as seen in the reactions outlined below.

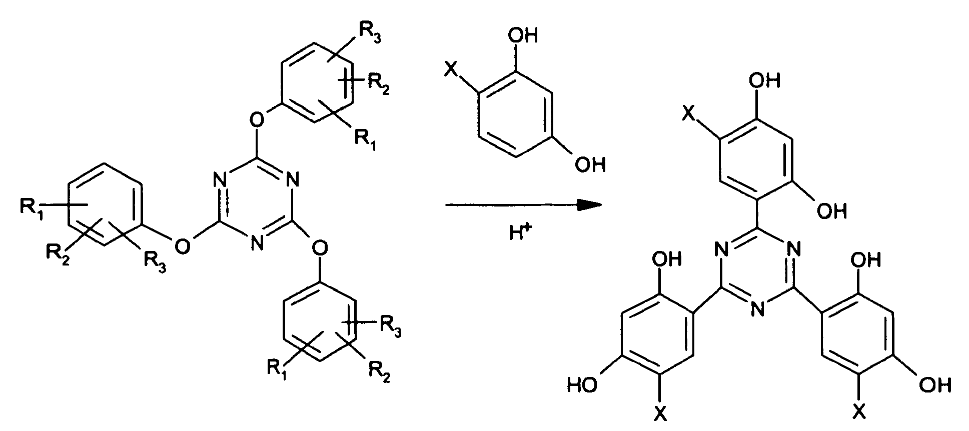

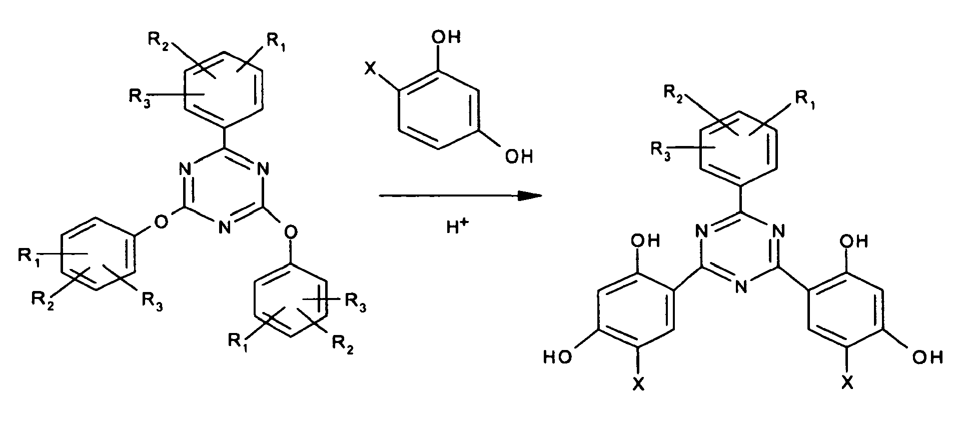

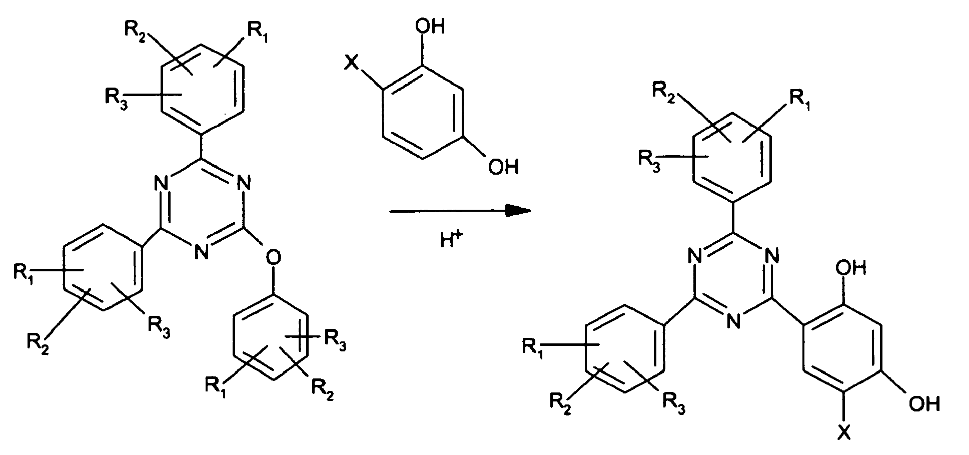

R₁, R₂ and R₃ are as defined above. The temperature ranges used are as above using chlorine as the leaving group.

The instant process also pertains to the preparation of asymmetric tris-aryl-s-triazines in only three steps while avoiding the selectivity problem of Friedel-Crafts reactions with cyanuric chloride. United States Patent Nos. 5,084,570 and 5,106,972 describe a four-step process to overcome this selectivity problem. The key to the new three step process is to take advantage of a nucleophilic substitution step on cyanuric chloride which is selective (E. M. Smolin et al., loc cit). The nucleophile introduced into the triazine ring will also perform as a leaving group under Friedel-Crafts conditions. It is a poor leaving group compared to chlorine. In this way selectivity under Friedel-Crafts condition is achieved. This allows one to avoid having to rechlorinate the triazine ring prior to subsequent Friedel-Crafts reactions thus reducing the number of synthetic steps. Effective nucleophiles which also serve as Friedel-Crafts leaving groups are alkoxy and aryloxy groups. Especially effective are phenoxy and substituted phenoxy.

For example, in order to prepare a mono-resorcinol-bis-aryl-s-triazine, cyanuric chloride is reacted with one mole of phenol or substituted phenol under nucleophilic (basic) conditions. 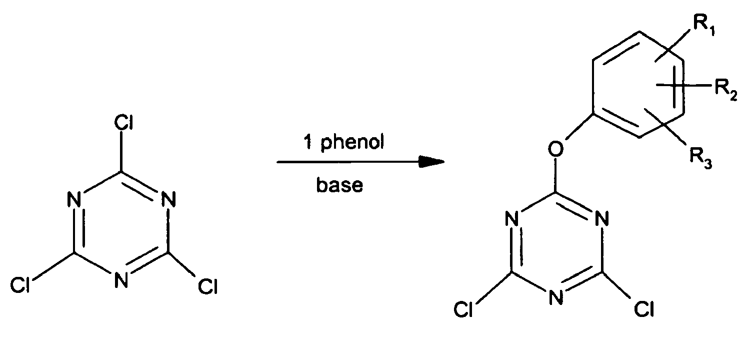 R₁, R₂ and R₃ are defined as above.

As mentioned above, the phenoxy group is an active Friedel-Crafts leaving group. It is a weaker one than chlorine thus allowing for the introduction of a relatively weak Friedel-Crafts substrate such as m-xylene, toluene, benzene, chlorobenzene or biphenyl while leaving the phenol group intact.

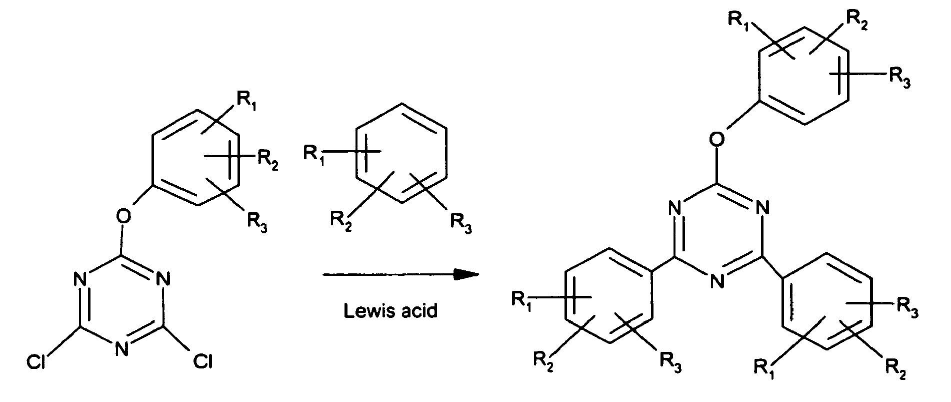

In the third step, the phenoxide may be replaced with a strong Friedel-Crafts substrate such as resorcinol. 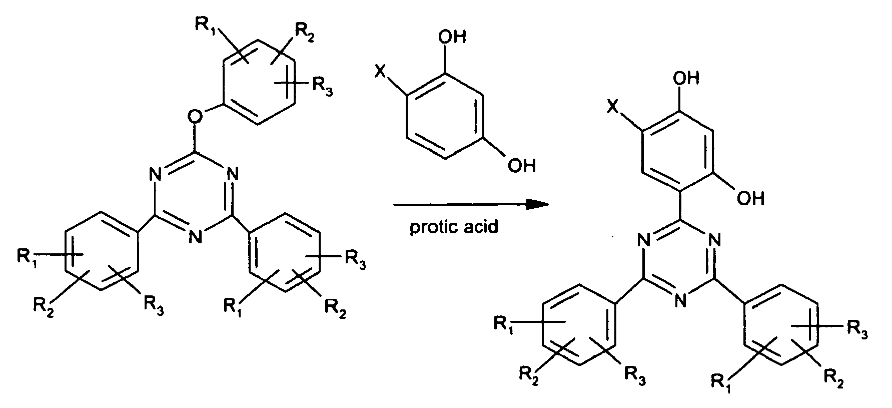 X, R₁, R₂ and R₃ are as defined above.

Japanese Hei 9-59263 discloses this three-step synthetic approach for the preparation of asymmetric tris-aryl-hydroxyphenyl-s-triazines. The preferred method of the Japanese reference is a one-pot process using a Lewis acid to mediate all three steps. The Japanese reference does not describe that step 3 can be mediated successfully by protic acids and that resorcinol itself may be used as a blocking group as is discussed further infra. This reference does not suggest the general applicability of the three-step synthesis.

Likewise, asymmetric tris-aryl-s-triazines which contain one weak and two strong Friedel-Crafts substrates may be prepared in three steps from cyanuric chloride. Examples are bis-resorcinol-mono-aryl-s-triazines where the aryl group is m-xylene, toluene, benzene, chlorobenzene or biphenyl. These materials are prepared by reacting cyanuric chloride with two moles of a phenol or substituted phenol under basic conditions to form a mono-chloro-bis-phenoxy-s-triazine. The remaining chlorine atom may be replaced with a weak Friedel-Crafts substrate such as m-xylene, toluene, benzene, chlorobenzene or biphenyl leaving the phenoxy groups intact. The phenoxy groups may then be replaced by a strong Friedel-Crafts substrate such as resorcinol.

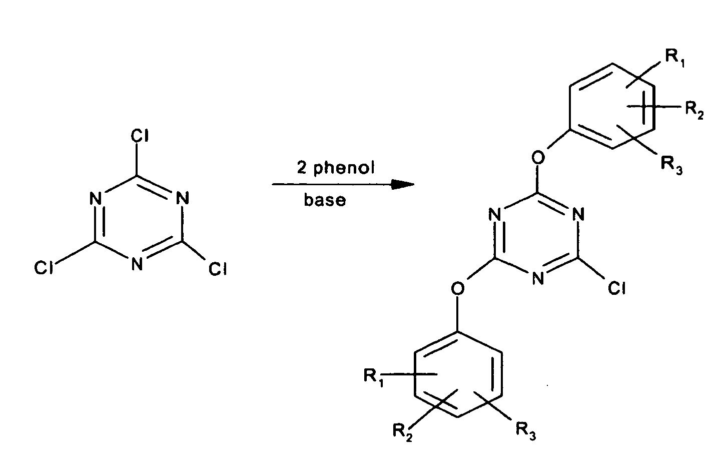

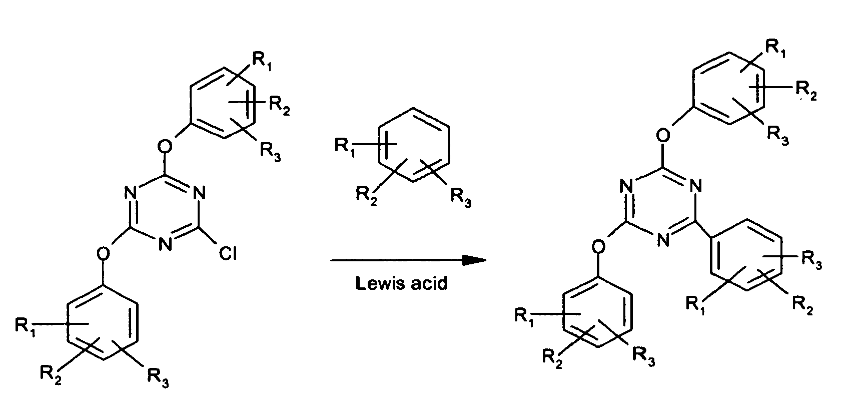

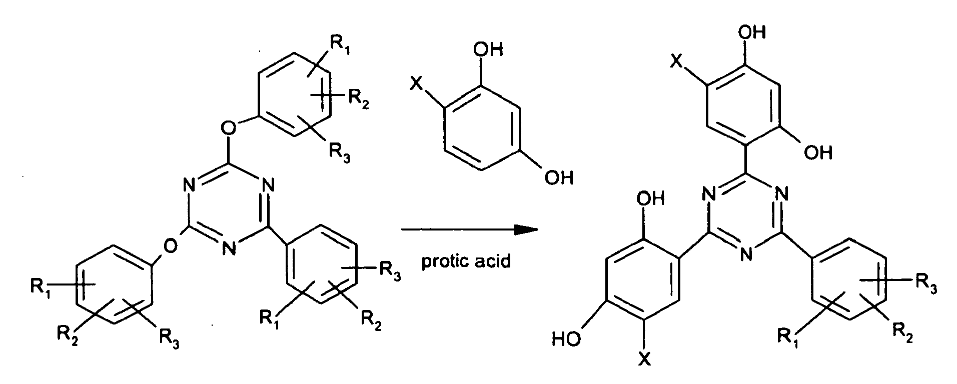

A special embodiment of this approach to prepare mono-resorcinol-bis-aryl-s-triazine would be to use resorcinol itself as the blocking phenol in step 1. A bis-triazine-resorcinol adduct is formed and is carried through steps 2 and 3 as seen above. This method is especially advantageous if excess resorcinol is used. In an industrial process only one phenol (namely resorcinol) would need to be recovered and recycled instead of two thus allowing for substantial cost savings. This embodiment is outlined below.

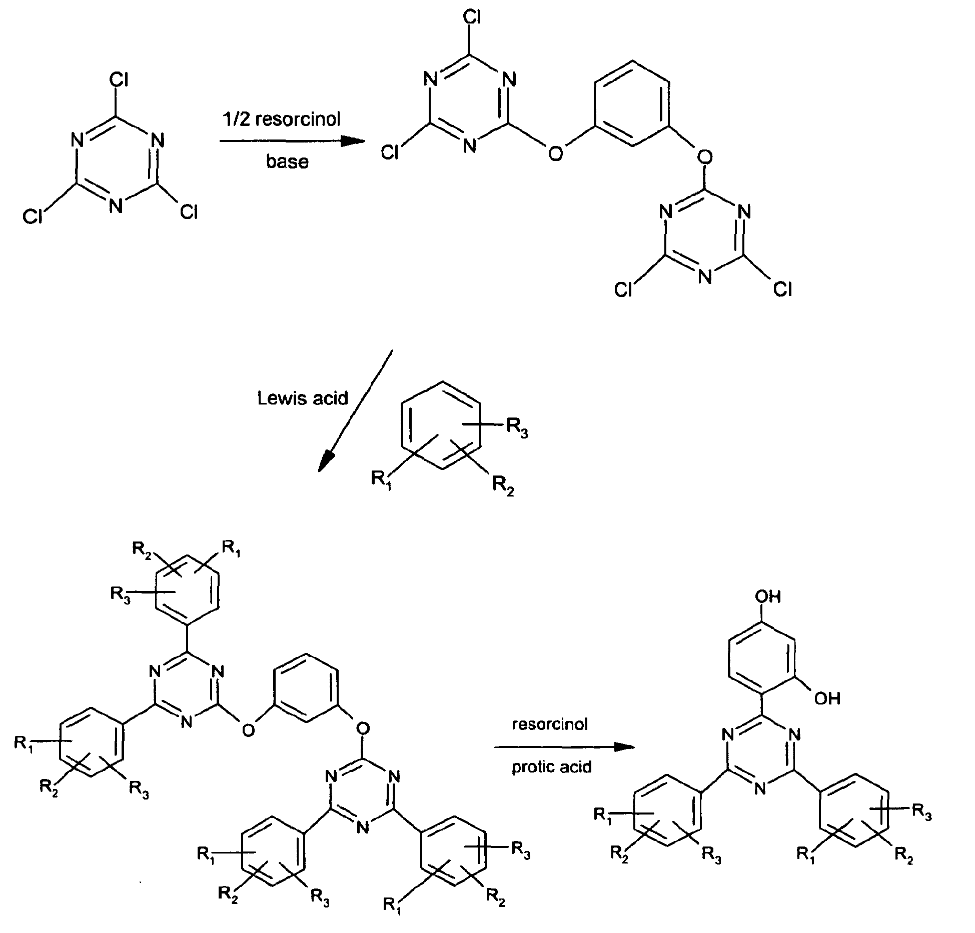

The nucleophilic first step in the special embodiment above may be performed in a variety of solvents such as acetone, acetone/water mixtures and xylene. It may also be carried out by means of a two-phase reaction with water and a hydrocarbon solvent such as xylene. A phase-transfer catalyst including quaternary ammonium salts or polyether such as glymes or poly(ethylene glycol) aids the reaction. Bases which can be used include hydroxide, carbonate and bicarbonate salts of sodium, potassium and calcium. Effective temperatures range from -20°C to 100°C. The most effective temperatures range from -20°C to 50°C.

Especially effective condition are the use of 10 % water in acetone as solvent and sodium hydroxide as the base at a temperature range of -20°C to 5°C. Also especially effective is the use of a two-phase system of water/xylene with sodium bicarbonate as the base and a quaternary ammonium salt such as benzyltrimethylammonium chloride as phase-transfer catalyst at temperatures of -5°C to 10°C. Also especially effective is the use of benzyltrimethylammonium chloride as a catalyst in xylene at temperatures of -10°C to 10°C with potassium carbonate base under anhydrous conditions.

The second step in which chlorine is displaced by the weaker Friedel-Crafts substrate requires a Lewis acid catalyst. Aluminum chloride is the Lewis acid of choice and may be used in the range of 0.2 to 1.5 equivalents per equivalent of s-triazine. The reaction is best performed neat using an excess of the Friedel-Crafts substrate as solvent. Effective amounts of neat Friedel-Crafts reactant range from 2 to 20 molar equivalents. Examples of solvents which may be employed in this way include m-xylene, toluene, benzene, chlorobenzene and biphenyl. An effective temperature range for this reaction is from 0°C to 170°C; especially from 70°C to 150°C.

It is especially effective to perform the first step in a hydrocarbon solvent which will also be the Friedel-Crafts reactant for step two. Step two may then be performed without isolation of the product of step one.

Lewis acids usable within present process are mainly those known in the art as Friedel-Crafts catalysts; examples are AlCl₃, FeCl₃, ZnCl₂, TiCl₄, SnCl₂; especially preferred is AlCl₃.

Step three, in which the phenol is replaced by a strong Friedel-Crafts substrate such as resorcinol, may be performed with a protic acid catalyst. The preferred protic acids are hydrogen chloride gas and methanesulfonic acid. The reaction may be performed in the neat molten resorcinol. The amount of resorcinol may range from 1.2 to 10 molar equivalents per phenoxy group being displaced. The most effective range is from 1.2 to 4 molar equivalents of resorcinol per phenoxy moiety. The reaction may be performed e.g. with a catalyst level of 0.33 to 1.5 molar equivalents per triazine. The temperature for the reaction may range from 25°C to 200°C. The most effective temperature range is from 120°C to 170°C.

Present invention may be carried out according to process variants A-F. The most general form corresponds to variants D, E and F, corresponding to a process for preparing a compound of formula A 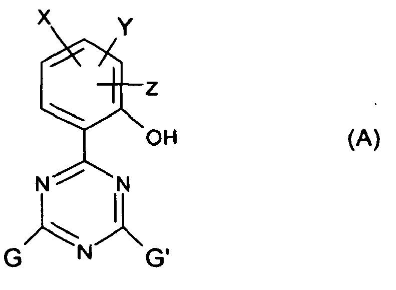 wherein
G and G' each independently are a residue of the formula 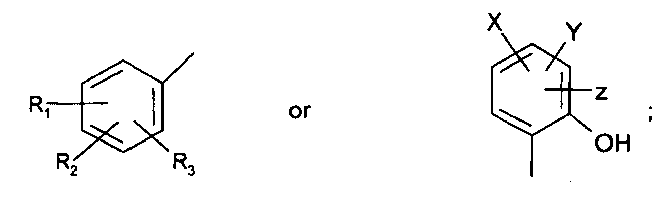
R₁, R₂ and R₃ are each independently hydrogen; alkyl of 1 to 12 carbon atoms; hydroxy; alkoxy of 1 to 12 carbon atoms; C₂-C₈alkenyl; C₃-C₁₈alkoxy interrupted by O and/or substituted by OH; C₂-C₁₈alkyl or C₂-C₁₈alkoxy, each interrupted by COO or substituted by COOH; halogen; nitro; amino; amino substituted by a C₁-C₁₂ hydrocarbon selected from alkyl, phenyl, cycloalkyl; C₂-C₁₈acylamino; C₂-C₁₂alkoxycarbonyl; C₅-C₁₂cycloalkyloxycarbonyl; C₇-C₁₅phenylalkoxycarbonyl; phenyl or phenyl substituted by alkyl of 1 to 12 carbon atoms, hydroxy, alkoxy of 1 to 12 carbon atoms or halogen; and
X, Y and Z, independently, are as defined for R₁, R₂ or R₃, or are phenylalkyl of 7 to 15 carbon atoms;
   which process comprises
   reacting a compound of the formula B 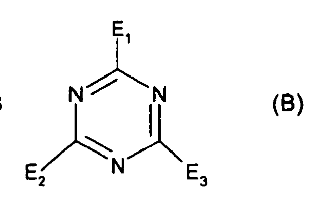 wherein
   E₁ is a leaving group selected from halogen, C₁-C₁₂alkoxy or 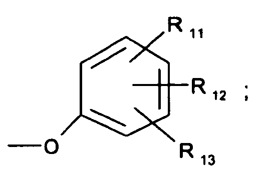 where R₁₁, R₁₂ and R₁₃ are as defined for R₁, R₂ and R₃, and R₁₁ also may be a residue of the formula 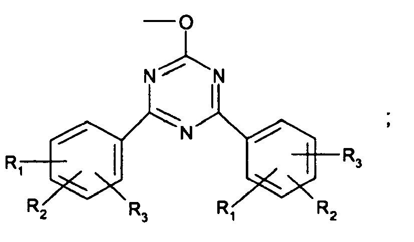 E₂ and E₃ are a leaving group as defined for E₁ or are G or G';
   with a compound of formula C 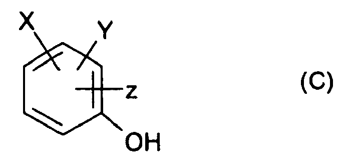 in the presence of an effective amount of a protic acid catalyst to give the compound of formula A.
   R₁, R₂, R₃ may also be SO₃H; SO₃⁻; CN; or R₁ and R₂, and especially X and Y, stand vicinal and form, together with the carbon atoms they are attached to, a carbocyclic ring of 5 or 6 carbon atoms, or a ring of 4 or 5 carbon atoms and -O-, -N= or -NH-.

Preferably, the protic acid is the only acid catalyst used, or it is used in combination with a Lewis acid potentiator, which is added in an amount of 0-0.25 equivalents Lewis acid per equivalent leaving group (such as chloride) or phenolic compound. If the leaving group is halogen, the amount of protic acid formed thereby can be sufficient so that no further addition of protic acid is needed. The total amount of protic acid is preferably 0.8 to about 20 equivalents, preferably about 1 to 2 equivalents per equivalent leaving group.

In process variants D and F, both G and G' a phenolic residue of the formula 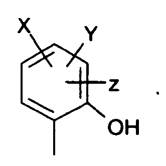
In the compounds of formulae A, B and C,
R₁, R₂ and R₃ preferably are each independently hydrogen; alkyl of 1 to 12 carbon atoms; alkoxy of 1 to 12 carbon atoms; C₂-C₈alkenyl; C₃-C₁₈alkoxy interrupted by O and/or substituted by OH; C₂-C₁₈alkyl or C₂-C₁₈alkoxy, each interrupted by COO or substituted by COOH; halogen; nitro; amino; amino substituted by a C₁-C₁₂ hydrocarbon selected from alkyl, phenyl, cycloalkyl; C₂-C₁₈acylamino; C₂-C₁₂alkoxycarbonyl; C₅-C₁₂cycloalkyloxycarbonyl; C₇-C₁₅phenylaikoxycarbonyl; phenyl or phenyl substituted by alkyl of 1 to 12 carbon atoms, hydroxy, alkoxy of 1 to 12 carbon atoms or halogen;
R₁₂ and R₁₃ are preferably as defined for X and Y, and
R₁₁ is preferably as defined for Z or R₁₁ also may be a residue of the formula 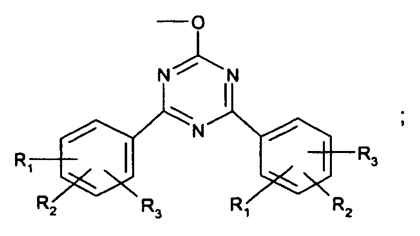
X and Y, independently, are preferably hydrogen; alkyl of 1 to 12 carbon atoms; phenylalkyl of 7 to 15 carbon atoms; and
Z is preferably in meta-position to OH and is hydroxy; alkoxy of 1 to 12 carbon atoms; C₃-C₁₈alkoxy interrupted by O and/or substituted by OH; C₂-C₁₈alkoxy interrupted by COO or substituted by COOH; halogen; nitro; amino; amino substituted by a C₁-C₁₂ hydrocarbon selected from alkyl, phenyl, cycloalkyl; C₂-C₁₈acylamino; C₂-C₁₂alkoxycarbonyl.

Most preferably, in the compounds of formulae A, B and C
X is hydrogen; alkyl of 1 to 12 carbon atoms; phenylalkyl of 7 to 15 carbon atoms or halogen;
Y is hydrogen;
Z is hydroxy;
R₁, R₂ and R₃ are each independently hydrogen; alkyl of 1 to 12 carbon atoms; hydroxy; alkoxy of 1 to 12 carbon atoms; halogen; phenyl or phenyl substituted by alkyl of 1 to 12 carbon atoms, hydroxy, alkoxy of 1 to 12 carbon atoms or halogen;
E₁, E₂ and/or E₃ as a leaving group in formula B are Cl.

Especially preferred are R₁, R₂ and R₃ each independently as hydrogen; alkyl of 1 to 4 carbon atoms; alkoxy of 1 to 12 carbon atoms; Cl; phenyl or phenyl substituted by alkyl of 1 to 4 carbon atoms, alkoxy of 1 to 12 carbon atoms;
R₁₂ and R₁₃ defined for X and Y, and
R₁₁ as defined for Z or R₁₁ is a residue of the formula

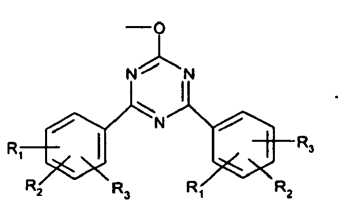

Accordingly, a preferred process leads to a compound of formula I 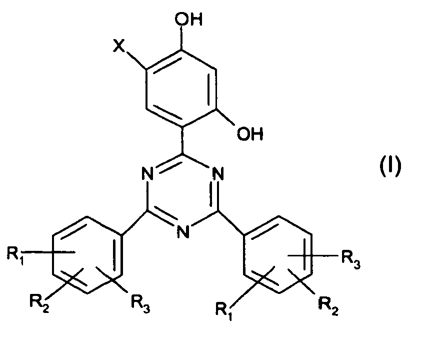 from a starting compound of formula XIII 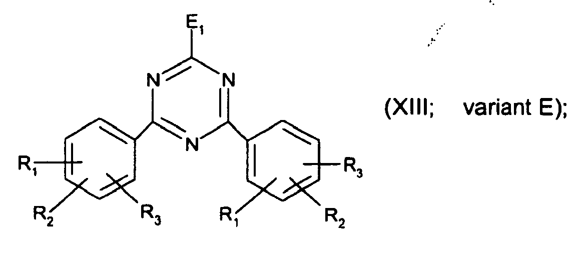 or to a compound of formula VII 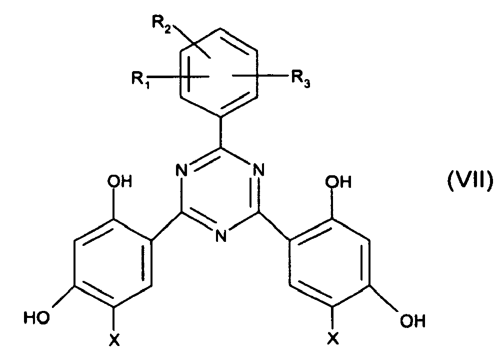 from the starting compound of formula XIV 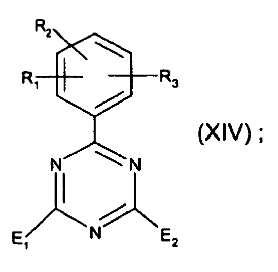 or to a compound of formula XII 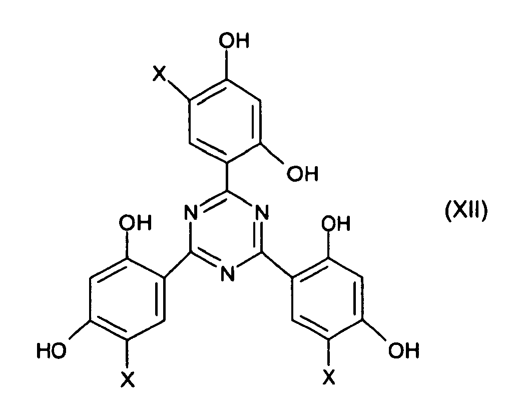 from the starting compound of the formula B 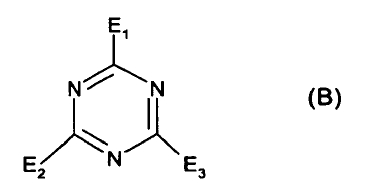 where E₁, E₂ and E₃ each is a leaving group selected from F, Cl and 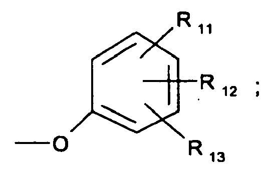 where R₁₁, R₁₂ and R₁₃ are as defined for R₁, R₂ and R₃, and R₁₁
also may be a residue of the formula 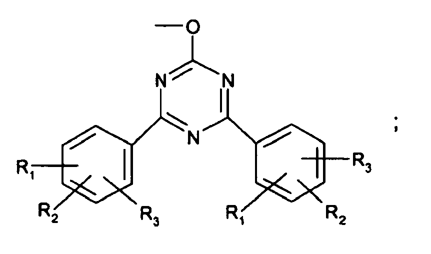
X is hydrogen; alkyl of 1 to 12 carbon atoms; phenylalkyl of 7 to 15 carbon atoms or halogen; and
R₁, R₂ and R₃ are each independently hydrogen; alkyl of 1 to 12 carbon atoms; hydroxy; alkoxy of 1 to 12 carbon atoms; halogen; phenyl or phenyl substituted by alkyl of 1 to 12 carbon atoms, hydroxy, alkoxy of 1 to 12 carbon atoms or halogen; and comprises reacting the starting compound with a resorcinol of formula VI 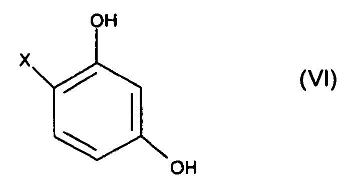 in the presence of an effective amount of a protic acid to form a compound of formula XII.

The amount of resorcinol of formula VI preferably is
one to ten equivalents per equivalent of the compound of formula XIII for the preparation of a compound of the formula I or
two to twenty equivalents per equivalent of the compound of formula XIV for the preparation of a compound of the formula VII or
three to thirty equivalents per equivalent of the compound of formula B for the preparation of a compound of the formula XII.

The amount of protic acid is usually 0.5 to 10, often 0.7 to 5, especially ca. 0.8 to 1.5 equivalents per equivalent of the formula C or VI; the protic acid is preferably a hydrogen halide, sulfuric or sulfonic acid, especially hydrogen chloride or methanesulfonic acid. The protic acid can also be used along with a Lewis acid potentiator at the level of less than 0.25, e.g. 0.01 to 0.2, equivalents of Lewis acid per equivalent of chloride formed.
The leaving group E₁, E₂ and E₃ is preferably Cl or F or 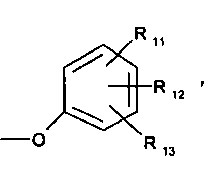 especially Cl; thus a preferred starting material of formula b is cyanuric chloride 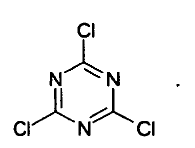 The process can be carried out in the melt or in a solvent, e.g. an aromatic hydrocarbon or tetramethylene sulfone. With the leaving group halogen, no extra acid catalyst is needed. Variants A, B and C of present process pertain to a 3 step process for preparing a compound of formula A 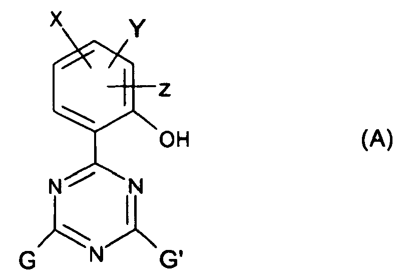 wherein
G is a residue of the formula 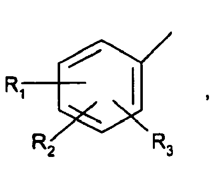 and
G' is a residue of the formula 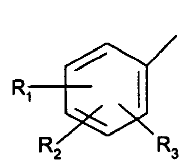 in process variant A and B or is a residue of the formula 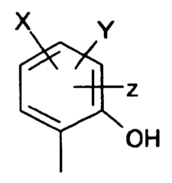 in process variant C;
R₁, R₂ and R₃ are each independently hydrogen; alkyl of 1 to 12 carbon atoms; hydroxy; alkoxy of 1 to 12 carbon atoms; C₂-C₈alkenyl; C₃-C₁₈alkoxy interrupted by O and/or substituted by OH; C₂-C₁₈alkyl or C₂-C₁₈alkoxy, each interrupted by COO or substituted by COOH; halogen; nitro; amino; amino substituted by a C₁-C₁₂ hydrocarbon selected from alkyl, phenyl, cycloalkyl; C₂-C₁₈acylamino; C₂-C₁₂alkoxycarbonyl; C₅-C₁₂cycloalkyloxycarbonyl; C₇-C₁₅phenylalkoxycarbonyl; phenyl or phenyl substituted by alkyl of 1 to 12 carbon atoms, hydroxy, alkoxy of 1 to 12 carbon atoms or halogen; and
X, Y and Z, independently, are as defined for R₁, R₂ or R₃, or are phenylalkyl of 7 to 15 carbon atoms; which process comprises
   (1.) reacting a compound of the formula 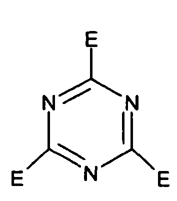 wherein
      E is halogen, with a phenolic compound of formula II 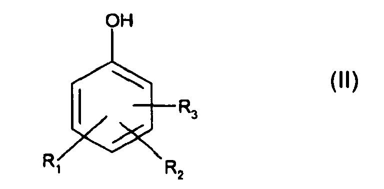 in the presence of a base to form a compound of formula III 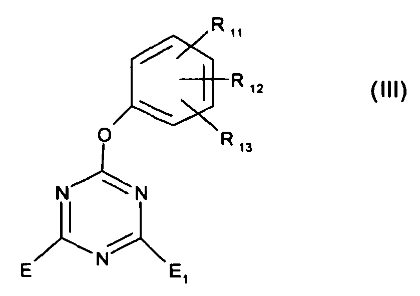 wherein R₁₁, R₁₂ and R₁₃, independently, are as defined for R₁, R₂ or R₃, and R₁₁ also may be
      a residue of the formula 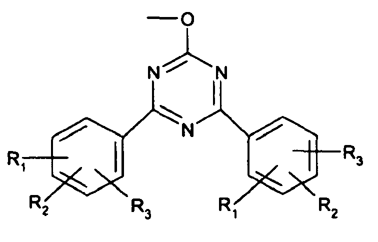 (variant B); and
      E₁ is halogen or a residue of the formula 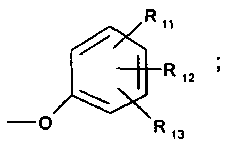
   (2). reacting the compound of formula III neat with a compound of formula IV 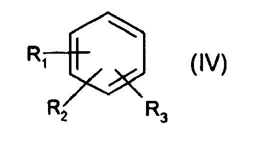 in the presence of an effective amount of a Lewis acid catalyst to give a compound of formula V 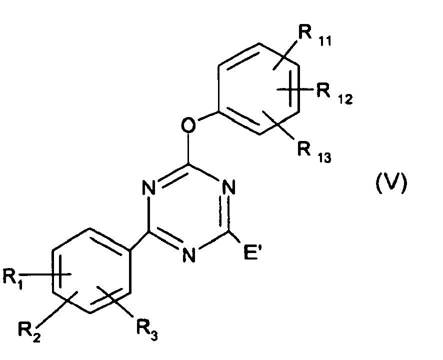 where E' is a residue of the formula 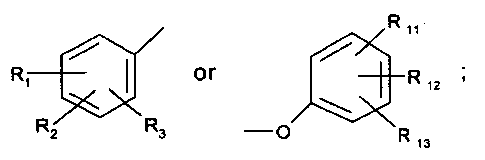
   (3). reacting the compound of formula V neat with a compound of formula VI 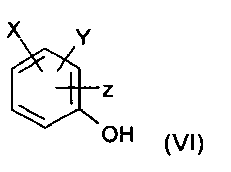 in the presence of an effective amount of a protic acid catalyst as the only catalyst to give the compound of formula A.

Preferably, when G' in formula A is a residue of the formula 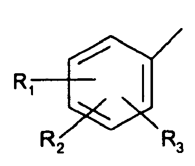 (variants A or B), step 1 is run at -20°C to 100°C, and at least two equivalents of the compound of formula IV are used in step 2, and 1.2 to ten equivalents of compound of formula VI are used per one equivalent of triazine in step 3, and when none of R₁, R₂ or R₃ in formula II is hydroxy, in step 1 one equivalent of the phenolic compound of formula II (variant A), and when one of R₁, R₂ or R₃ is hydroxy, in step 1 half an equivalent of the phenolic compound of formula II (variant B) is used to form a compound of formula III 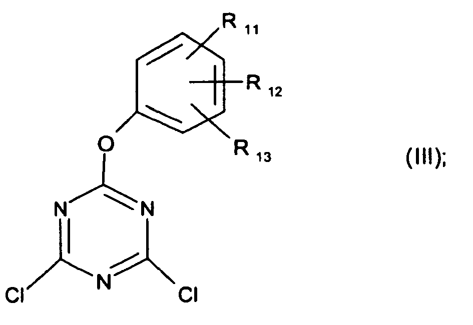 and when G' in formula A is a residue of the formula 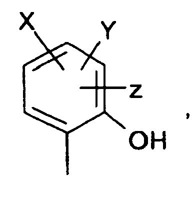 two equivalents of the phenolic compound of formula II used in step 1 to form a compound of formula VIII 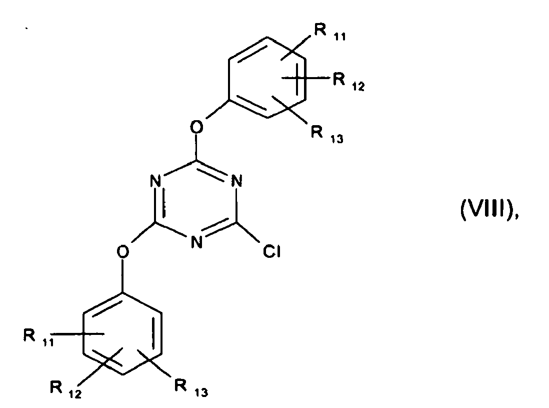 and step 1 run at -20°C to 200°C; at least one equivalent of the compound of formula IV is used in step 2, and 2.4 to twenty equivalents of compound of formula VI are used per one equivalent of triazine in step 3.

Especially preferred is a process for preparing a compound of formula A, wherein all 3 phenyl moieties bonded to the triazine ring are the same and are 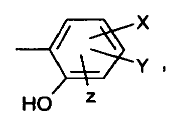 or wherein each of G and G' are 4-phenylphenyl or 4-phenylphenyl substituted by alkyl of 1 to 12 carbon atoms, alkoxy of 1 to 12 carbon atoms or halogen.
Especially preferred is a process wherein the phenolic compounds of formulae C, II and VI are identical and are of the formula 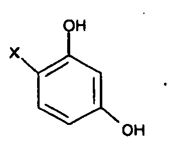 Also preferred is a process where the aromatic compound of the formula IV is not a phenol, i.e. none of R₁, R₂ and R₃ in formula IV is hydroxy; the compound of formula IV is especially preferred as a pure hydrocarbon of 6-20 carbon atoms.

More specifically, the process of present invention may be carried out according to the embodiments A, B, C, D, E or F as follows:
A is preferably a process for preparing a compound of formula I 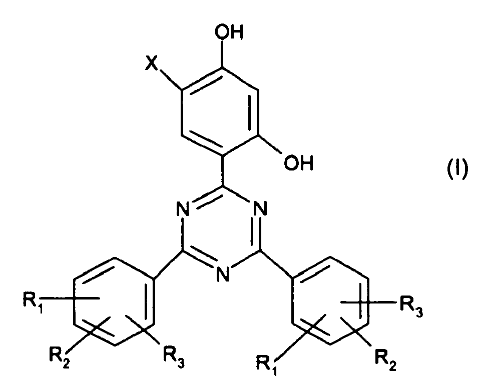 where
   X is hydrogen, alkyl of 1 to 12 carbon atoms, phenylalkyl of 7 to 15 carbon atoms or halogen, and
   R₁, R₂ and R₃ are each independently hydrogen, alkyl of 1 to 12 carbon atoms, hydroxy, alkoxy of 1 to 12 carbon atoms, halogen, phenyl or phenyl substituted by alkyl of 1 to 12 carbon atoms, hydroxy, alkoxy of 1 to 12 carbon atoms or halogen,
   which process comprises
   (1). reacting cyanuric chloride with a phenolic compound of formula II 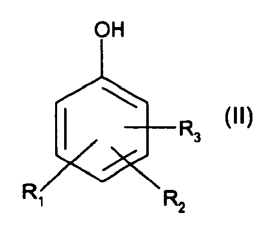 to form a compound of formula III 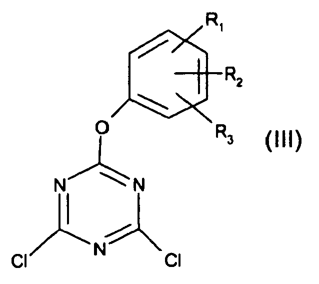
   (2). reacting the compound of formula III with an aromatic hydrocarbon of formula IV 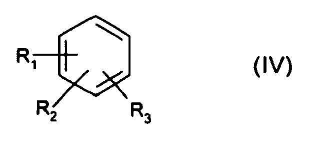 in the presence of an effective amount of a Lewis acid catalyst to give a compound of formula V 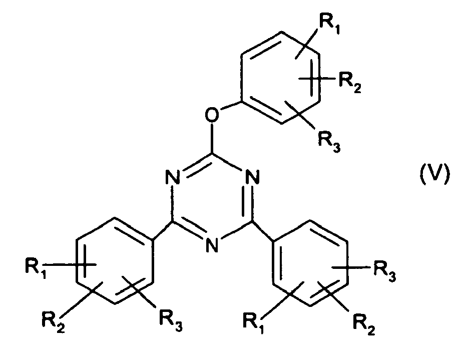
   (3). reacting the compound of formula V with a compound of formula VI 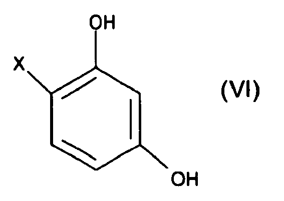 in the presence of an effective amount of a protic acid catalyst to give the compound of formula I.
B is preferably a process for preparing a compound of formula I 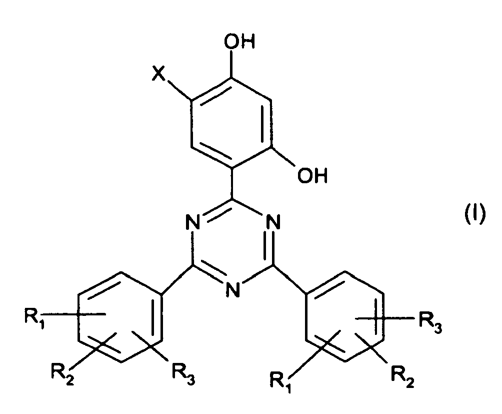 where
   X is hydrogen, alkyl of 1 to 12 carbon atoms, phenylalkyl of 7 to 15 carbon atoms or halogen, and
   R₁, R₂ and R₃ are each independently hydrogen, alkyl of 1 to 12 carbon atoms, hydroxy, alkoxy of 1 to 12 carbon atoms, halogen, phenyl or phenyl substituted by alkyl of 1 to 12 carbon atoms, hydroxy, alkoxy of 1 to 12 carbon atoms or halogen,
   which process comprises
   (1). reacting cyanuric chloride with a half equivalent of a resorcinol to form a compound of formula X 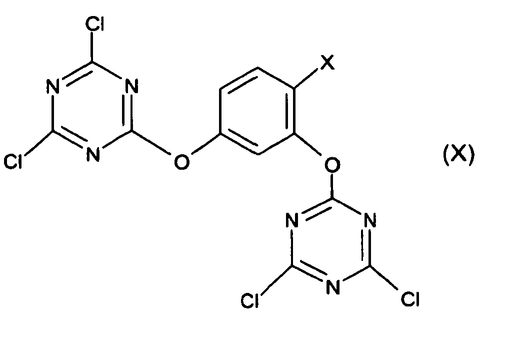
   (2). reacting the compound of formula X with an aromatic hydrocarbon of formula IV 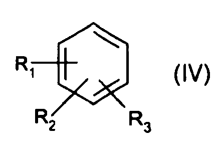 in the presence of an effective amount of a Lewis acid catalyst to give a compound of formula XI 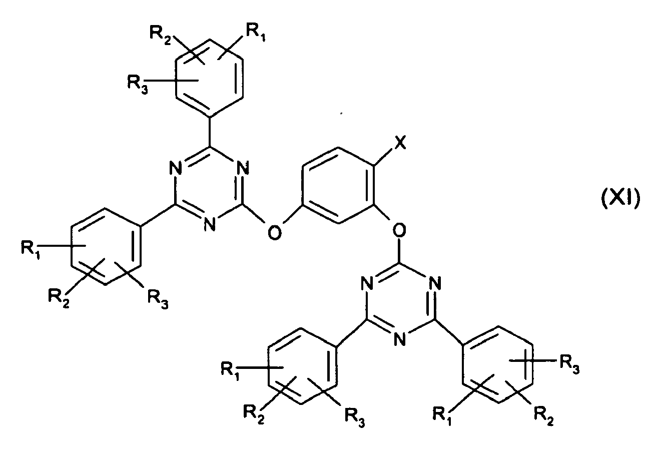
   (3). reacting the compound of formula XI with a compound of formula VI 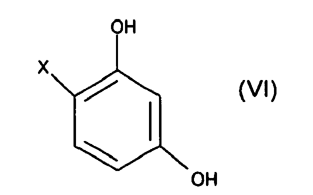 in the presence of an effective amount of a protic acid catalyst to give the compound of formula I.
C is preferably a process for preparing a compound of formula VII 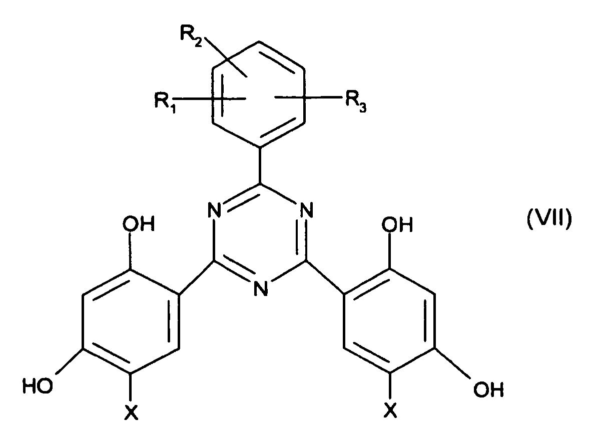 where
   X is hydrogen, alkyl of 1 to 12 carbon atoms, phenylalkyl of 7 to 15 carbon atoms or halogen, and
   R₁, R₂ and R₃ are each independently hydrogen, alkyl of 1 to 12 carbon atoms, hydroxy, alkoxy of 1 to 12 carbon atoms, halogen, phenyl or phenyl substituted by alkyl of 1 to 12 carbon atoms, hydroxy, alkoxy of 1 to 12 carbon atoms or halogen,
   which process comprises
   (1). reacting cyanuric chloride with two equivalents of a phenolic compound of the formula II 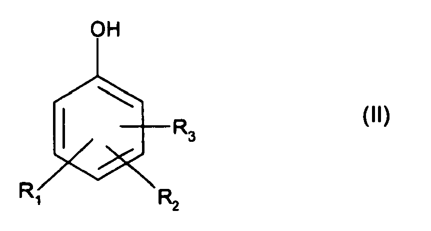 to form a compound of formula VIII 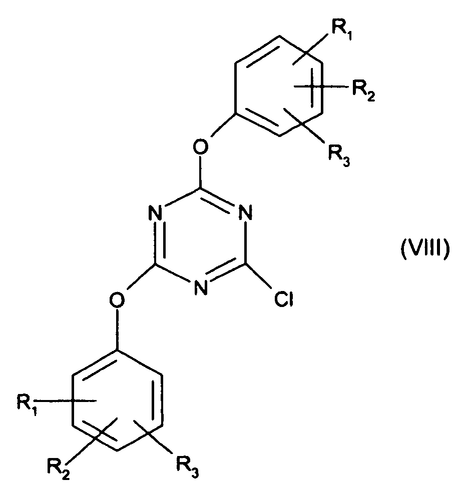
   (2). reacting the compound of formula VIII with an aromatic hydrocarbon of formula IV 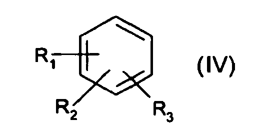 in the presence of an effective amount of a Lewis acid catalyst to give a compound of formula IX 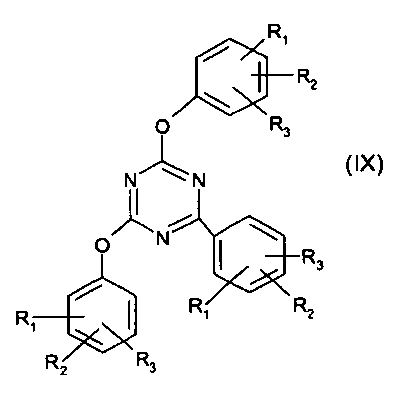
   (3). reacting the compound of formula IX with a compound of formula VI 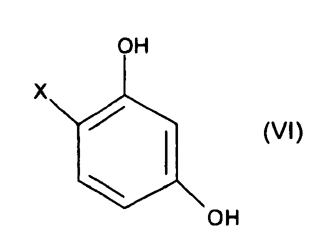 in the presence of an effective amount of a protic acid catalyst to give the compound of formula VII.

In process variants A, B and C, step 1 is carried out in the presence of a base, said base is preferably a hydroxide, carbonate or bicarbonate of sodium, potassium or calcium. most preferably sodium hydroxide, sodium bicarbonate or potassium carbonate.

In process variants A, B and C, step 1 is preferably run in acetone, acetone/water or a hydrocarbon. Another embodiment is when step 1 is run in a two-phase system of water and a hydrocarbon solvent, preferably xylene and where the phase transfer agent is a quaternary ammonium salt, a polyether or a poly(ethylene glycol), most preferably a quaternary ammonium salt, which is benzyltrimethylammonium chloride. Another embodiment is when step 1 is run in xylene with a phase transfer catalyst and potassium carbonate base under anhydrous conditions.

In process variants A and B, step 1 is usually run at -20°C to 100°C; preferably at - 20°C to 50°C.

In process variant C, step 1 is usually run at -20°C to 200°C; preferably at -20°C to 130°C.

In process variant A and B, step 1 is preferably run in 10 % water in acetone with sodium hydroxide as base at a temperature of -20°C to 5°C.

In process variant C, step 1 is preferably run in 10 % water in acetone with sodium hydroxide as base at a temperature of -20°C to 60°C.

In another embodiment of process variants A and B, step 1 is run in a two-phase system of aromatic hydrocarbon/water with sodium bicarbonate as the base and benzyltrimethylammonium chloride as a phase transfer catalyst at a temperature of -5°C to 10°C.

In another embodiment of process variant C, step 1 is run in a two-phase system of aromatic hydrocarbon/water with sodium bicarbonate as the base and benzyltrimethylammonium chloride as phase transfer catalyst at a temperature of -5°C to 110°C.

In still another embodiment of process variants A and B, step 1 is run in xylene with benzyltrimethylammonium chloride as catalyst and potassium carbonate as base under anhydrous conditions at a temperature of -10°C to 10°C.

In another embodiment of process variant C, step 1 is run in xylene with benzyltrimethylammonium chloride as catalyst and potassium carbonate as base under anhydrous conditions at a temperature of -10°C to 110°C.

Still another embodiment of process variants A, B and C, the product of step 1 is not isolated.

In process variants A, B and C, in step 2 the Lewis acid usually is aluminum chloride, preferably wherein the aluminum chloride is used in the range of 0.2 to 1.5 equivalents per one equivalent of s-triazine.

In process variants A, B and C, step 2 is usually run at a temperature of 0°C to 170°C; especially 70°C to 150°C.

In process variants A, B and C, step 2 is conveniently run neat in an excess of aromatic compound of formula IV, preferably where the aromatic compound of formula IV is m-xylene, toluene, benzene, chlorobenzene or biphenyl.

In process variants A, B and C, the compound of formula IV is often used in the range of from 2 to 20 molar equivalents per equivalent of triazine of the compound of formula III, X or VIII.

In process variants A, B and C, compounds of formulae III, X or VIII conveniently are not isolated, the Lewis acid is added and the temperature increased, if necessary, to produce the compound of formula V, XI or IX.

In process variants A, B and C, in step 3 usually a protic acid is used, which is preferably hydrogen chloride gas or methanesulfonic acid.

In process variants A, B and C, step 3 is conveniently carried out using 1.2 to ten equivalents of the compound of formula C or VI, most preferably a resorcinol, per each phenoxy-triazine bond.

In process variants A, B and C, step 3 is usually carried out at a temperature of from 25°C to 200°C, preferably from 120°C to 170°C.

In process variants A, B and C, in step 3, four equivalents of C or VI, especially a resorcinol, and 1 to 1.5 equivalents of methanesulfonic acid are preferably used per each phenoxy-triazine bond; or four to ten equivalents of C or VI, especially resorcinol, are used per each phenoxy-triazine bond; or 1.2 to 1.5 equivalents of compounds of formulae C or VI, especially resorcinol, and 0.8 to 1.5 equivalents of Lewis acid are used per each phenoxy-triazine bond.

In process variants A, B and C, step 3 conveniently may be performed neat.

D is preferably a process for preparing a compound of formula XII 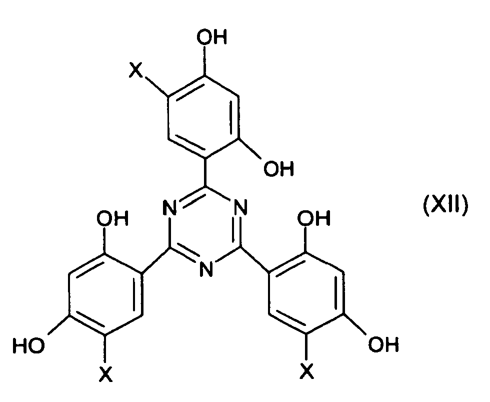 where
X is hydrogen, alkyl of 1 to 12 carbon atoms, phenylalkyl of 7 to 15 carbon atoms or halogen,
which process comprises
reacting cyanuric chloride with a resorcinol of formula VI 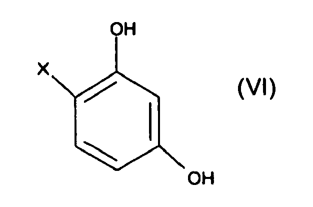 in the presence of an effective amount of a protic acid to form a compound of formula XII.
E is preferably a process for the preparing a compound of formula I 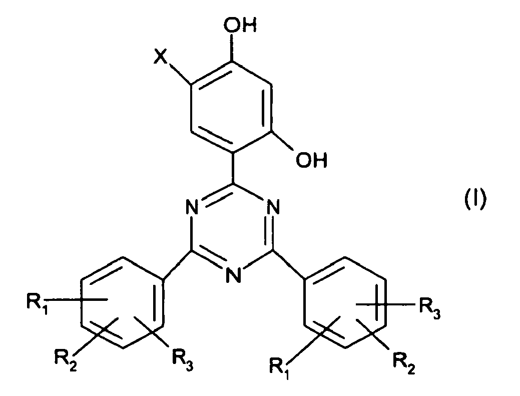 where
X is hydrogen, alkyl of 1 to 12 carbon atoms, phenylalkyl of 7 to 15 carbon atoms or halogen, and
R₁, R₂ and R₃ are each independently hydrogen, alkyl of 1 to 12 carbon atoms, hydroxy, alkoxy of 1 to 12 carbon atoms, halogen, phenyl or phenyl substituted by alkyl of 1 to 12 carbon atoms, hydroxy, alkoxy of 1 to 12 carbon atoms or halogen,
which process comprises reacting a compound of formula XIII 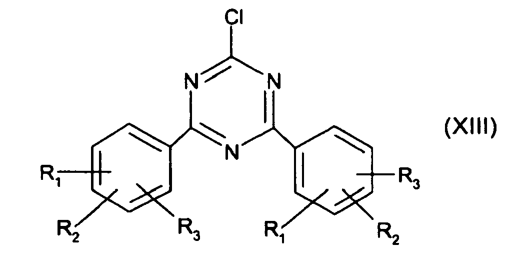 with a compound of formula VI 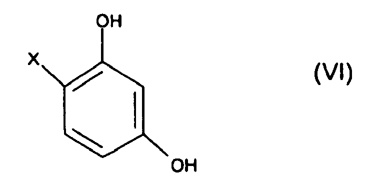 in the presence of an effective amount of a protic acid to form a compound of formula I.
F is preferably a process for the preparing a compound of formula XII 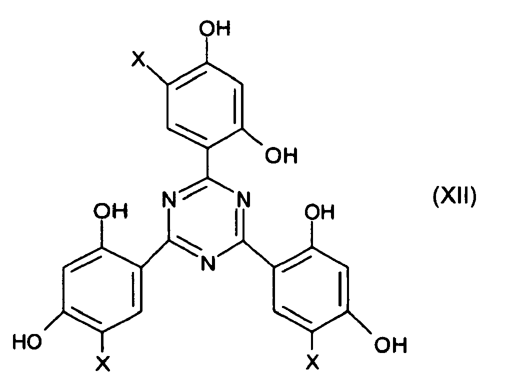 where
X is hydrogen, alkyl of 1 to 12 carbon atoms, phenylalkyl of 7 to 15 carbon atoms or halogen,
which comprises reacting a compound of formula XV 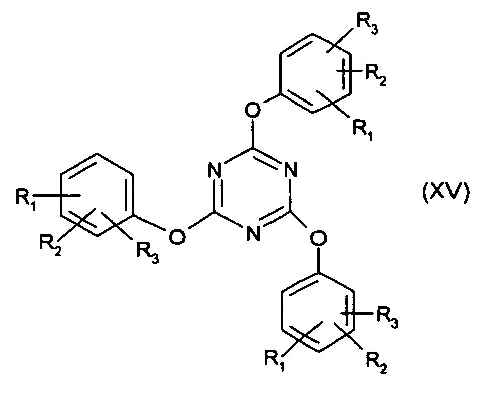 where
R₁, R₂ and R₃ are each independently hydrogen, alkyl of 1 to 12 carbon atoms, hydroxy, alkoxy of 1 to 12 carbon atoms, halogen, phenyl or phenyl substituted by alkyl of 1 to 12 carbon atoms, hydroxy, alkoxy of 1 to 12 carbon atoms or halogen,
with a compound of formula (VI) 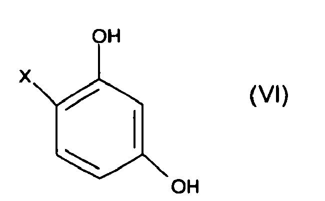 in the presence of an effective amount of a protic acid to form a compound of formula XII.
Further compounds obtainable according to present process correspond, for example, to the following formulae (R denoting H or C₁-C₈alkoxy, and X, R₁, R₂ and R₃ are as defined):

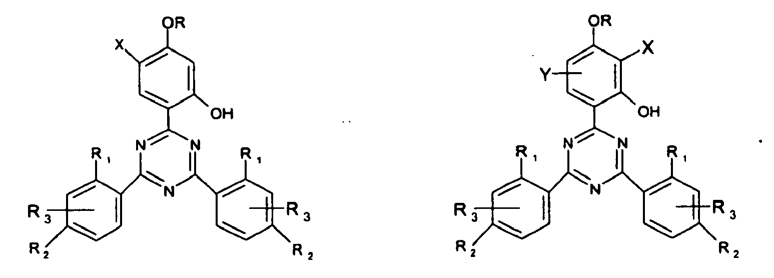

In process variants D, E and F, the reaction is carried out in the melt.

In process variants D and E, often no catalyst is added and 1.2 to ten equivalents of the compound of formula VI, especially resorcinol, are used per equivalent of chloride.

In process variants D, E and F, often a protic acid catalyst is added which is preferably hydrogen chloride or methanesulfonic acid.

In process variants D, E and F, the protic acid catalyst may also be used along with a Lewis acid potentiator at a level of less than 0.25 equivalents of Lewis acid per equivalent of leaving group or phenol.

In process variants D, E and F, the reaction is usually carried out at a temperature of from 70°C to 200°C; and most preferably at 100°C to 170°C.

### Example 1

### 2,4,6-Tris-(2,4-dihydroxyphenyl-s-triazine

To a 250 mL round-bottomed flask equipped with a condenser, magnetic stirrer and a nitrogen atmosphere is charged 5.60 g (0.0510 mol) of resorcinol. The stirred resorcinol is heated to 120°C at which point it becomes molten. A 2.08 g (0.0113 mol) portion of cyanuric chloride is added all at once resulting in an immediate evolution of hydrogen chloride gas. After 30 minutes, a portion of water is added and the mixture is refluxed for two hours and then allowed to cool to room temperature. The crude yellow powder (3.52 g) is collected by vacuum filtration. ¹H nmr (DMSO-d₆) reveals an overall conversion of 50 % to the title compound.

### Alternatively:

To a 250 mL round-bottomed flask equipped with a condenser, magnetic stirrer and a nitrogen atmosphere is added 1.00 g (2.80 mmol) of cyanuric chloride, 5.00 g (45.5 mmol) of resorcinol and 0.21 g (2.2 mmol) of methanesulfonic acid. The molten mixture is stirred at 130°C for 2.5 hours. The mixture is then triturated with hot water and 0.410 g of a brown-red solid is collected by vacuum filtration. ¹H nmr (DMSO-d₆) reveals the presence of the title compound.

### Example 2

### 4,6-Bis-(2,4-dimethylphenyl)-2-phenoxy-s-triazine

To a 1 L four-necked, round-bottomed flask equipped with a mechanical stirrer and an addition funnel is charges 37.5 g (0.203 mol) of cyanuric chloride and 200 mL of m-xylene. The suspension/solution is chilled below 5°C and 34.5 g of sodium bicarbonate, 200 mL of water and 1.4 g of benzyltrimethylammonium chloride are added. The contents of the flask are again cooled below 5°C. A solution of phenol (19.2 g, 0.203 mol) in 100 mL of m-xylene is charged to the addition funnel and then added over a 10-minute period to the reaction flask while the temperature is maintained between 1°C and 3°C. The mixture is then stirred at 2-7°C for five hours.

The mixture is then warmed to 50°C and the phases are separated. The organic phases is returned to the reaction flask and 32.5 g (0.244 mol) of aluminum chloride are added at 35°C. The mixture is then heated at about 130°C for three hours. The contents are cooled below 120°C and poured into 500 mL of cold 2N hydrochloric acid. The phases are separated and the organic phases is washed twice with water and one with saturated sodium bicarbonate solution. After drying over anhydrous potassium carbonate, the solvent is removed under reduced pressure to afford an oil which crystallized upon cooling. The crude solid is recrystallized from isopropanol to afford 57.7 g (75 % yield) of the title compounds melting at 96-98°C.

Examples 3 and 4 below represent some of the various novel methods which can be used to prepare 4,6-bis-(2,4-dimethylphenyl)-2-(2,4-dihydroxyphenyl)-s-triazine.

### Example 3

### 4,6-Bis-(2,4-dimethylphenyl)-2-(2,4-dihydroxyphenyl)-s-triazine

To a 50 mL round-bottomed flask equipped with a magnetic stirrer, condenser and nitrogen atmosphere are charged 1.00 g (3.10 mmol) of 4,6-bis-(2,4-dimethylphenyl)-2-chloro-s-triazine and 1.70 g (15.5 mmol) of resorcinol. The mixture is stirred at 180°C for five hours at which point ¹H nmr (CDCl₃) of an aliquot reveals a 72 % overall conversion to the title compound.

### Example 4

### 4,6-Bis-(2,4-dimethylphenyl)-2-(2,4-dihydroxyphenyl)-s-triazine

A 300 mL three-necked, round-bottomed flask fitted with a magnetic stirrer, an acid trap, a gas inlet and a heat lamp to prevent sublimation is charged with 29.9 g (0.272 mol) of resorcinol and 2.00 g (0.00525 mol) of the product of Example 2. The mixture is heated to 150°C and hydrogen chloride gas is bubbled slowly through the molten mixture for 25 minutes. The mixture is stirred at this temperature for another 4.5 hours and then allowed to cool to room temperature. Portions of water and toluene are added and the mixture is refluxed till the solids are dissolved. The mixture is allowed to cool to room temperature and the layers separated. The organic layer is washed once with water, twice with saturated sodium bicarbonate solution and once with brine. After drying over anhydrous magnesium sulfate and filtering, the solvent is removed under reduced pressure to afford 1.56 g of crude product. Purification by flash chromatography on silica gel with 3:1 heptane:ethyl acetate gives 1.19 g (57 % yield) of the title compound as a yellow solid.

## Claims

1. A process for preparing a compound of formula A 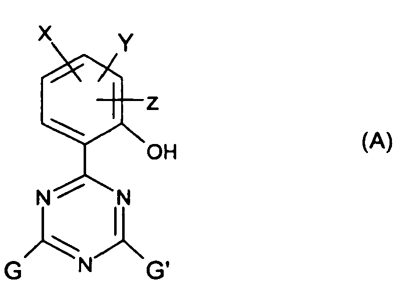 wherein
G and G' each independently are a residue of the formula 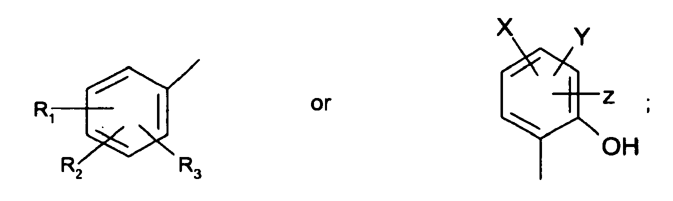
R₁, R₂ and R₃ are each independently hydrogen; alkyl of 1 to 12 carbon atoms; hydroxy; alkoxy of 1 to 12 carbon atoms; C₂-C₈alkenyl; C₃-C₁₈alkoxy interrupted by O and/or substituted by OH; C₂-C₁₈alkyl or C₂-C₁₈alkoxy, each interrupted by COO or substituted by COOH; halogen; nitro; SO₃H; SO₃⁻; CN; amino; amino substituted by a C₁-C₁₂ hydrocarbon selected from alkyl, phenyl, cycloalkyl; C₂-C₁₈acylamino; C₂-C₁₂alkoxycarbonyl; C₅-C₁₂cycloalkyloxy-carbonyl; C₇-C₁₅phenylalkoxycarbonyl; phenyl or phenyl substituted by alkyl of 1 to 12 carbon atoms, hydroxy, alkoxy of 1 to 12 carbon atoms or halogen; or R₁ and R₂ stand vicinal and form, together with the carbon atoms they are attached to, a carbocyclic ring of 5 or 6 carbon atoms, or a ring of 4 or 5 carbon atoms and -O-, -N= or -NH-; and
X, Y and Z, independently, are as defined for R₁, R₂ or R₃, or are phenylalkyl of 7 to 15 carbon atoms;
which process comprises
reacting a compound of the formula B 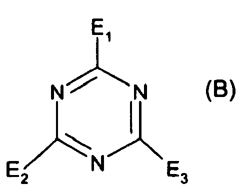 wherein
E₁ is a leaving group selected from halogen, C₁-C₁₂alkoxy or 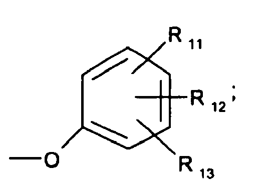 where R₁₁, R₁₂ and R₁₃ are as defined for R₁, R₂ and R₃, and R₁₁ also may be a residue of the formula 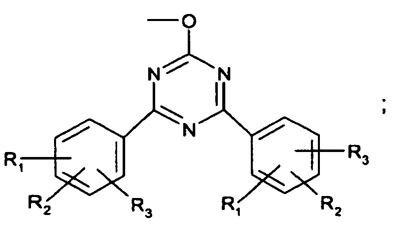 E₂ and E₃ are a leaving group as defined for E₁ or are G or G';
with a compound of formula C 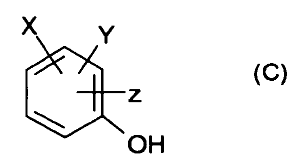 in the presence of an effective amount of a protic acid catalyst to give the compound of formula A, whereby the reaction is carried out in the melt.

2. Process of claim 1, where in the compounds of formulae A, B and C
R₁, R₂ and R₃ are each independently hydrogen; alkyl of 1 to 12 carbon atoms; alkoxy of 1 to 12 carbon atoms; C₂-C₈alkenyl; C₃-C₁₈alkoxy interrupted by O and/or substituted by OH; C₂-C₁₈alkyl or C₂-C₁₈alkoxy, each interrupted by COO or substituted by COOH; halogen; nitro; amino; amino substituted by a C₁-C₁₂ hydrocarbon selected from alkyl, phenyl, cycloalkyl; C₂-C₁₈acylamino; C₂-C₁₂alkoxycarbonyl; C₅-C₁₂cycloalkyloxy-carbonyl; C₇-C₁₅phenylalkoxycarbonyl; phenyl or phenyl substituted by alkyl of 1 to 12 carbon atoms, hydroxy, alkoxy of 1 to 12 carbon atoms or halogen;
R₁₂ and R₁₃ are as defined for X and Y, and
R₁₁ is as defined for Z or R₁₁ also may be a residue of the formula 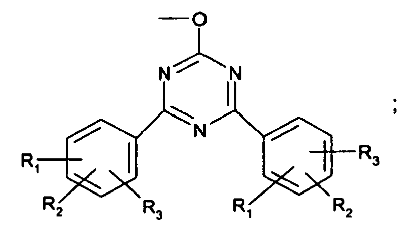 X and Y, independently, are hydrogen; alkyl of 1 to 12 carbon atoms; phenylalkyl of 7 to 15 carbon atoms; and
Z is in meta-position to OH and is hydroxy; alkoxy of 1 to 12 carbon atoms; C₃-C₁₈alkoxy interrupted by O and/or substituted by OH; C₂-C₁₈alkoxy interrupted by COO or substituted by COOH; halogen; nitro; amino; amino substituted by a C₁-C₁₂ hydrocarbon selected from alkyl, phenyl, cycloalkyl; C₂-C₁₈acylamino; C₂-C₁₂alkoxycarbonyl.

3. Process of claim 2, where in the compounds of formulae A, B and C
X is hydrogen; alkyl of 1 to 12 carbon atoms; phenylalkyl of 7 to 15 carbon atoms or halogen;
Y is hydrogen;
Z is hydroxy;
R₁, R₂ and R₃ are each independently hydrogen; alkyl of 1 to 12 carbon atoms; hydroxy; alkoxy of 1 to 12 carbon atoms; halogen; phenyl or phenyl substituted by alkyl of 1 to 12 carbon atoms, hydroxy, alkoxy of 1 to 12 carbon atoms or halogen;
E₁, E₂ and/or E₃ as a leaving group in formula B are Cl.

4. A process according to claim 1 for preparing a compound of formula I 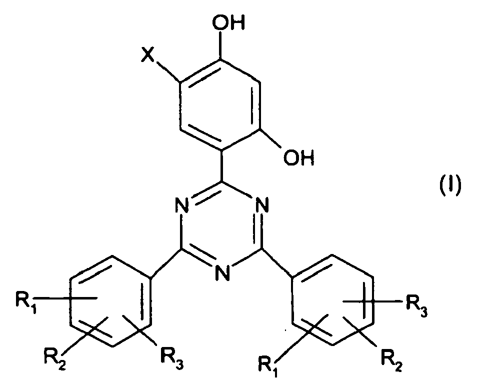 from a starting compound of formula XIII 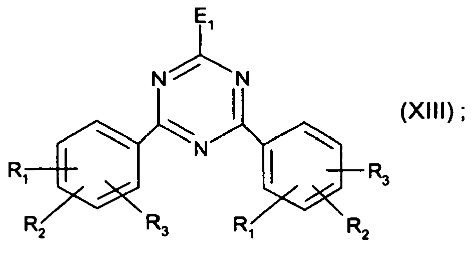 or a compound of formula VII 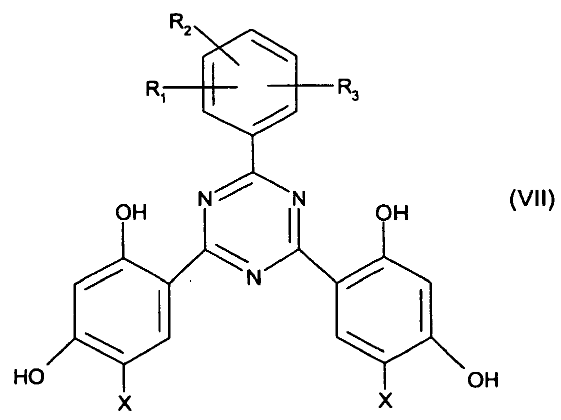 from the starting compound of formula XIV 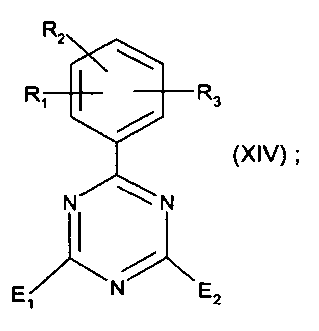 or a compound of formula XII 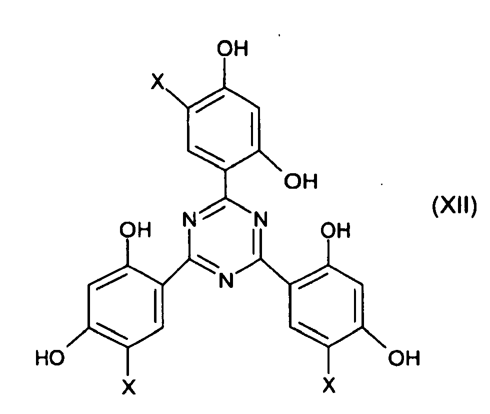 from the starting compound of the formula B 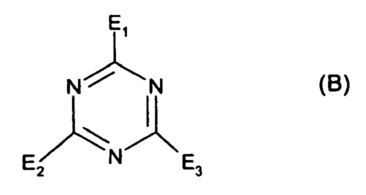 where
E₁, E₂ and E₃ each is a leaving group selected from F, Cl and 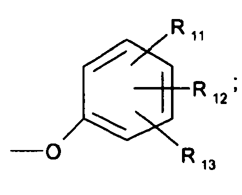 where R₁₁, R₁₂ and R₁₃ are as defined for R₁, R₂ and R₃, and R₁₁ also may be a residue of the formula 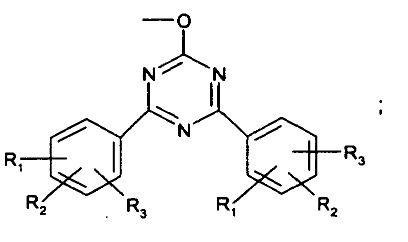
X is hydrogen; alkyl of 1 to 12 carbon atoms; phenylalkyl of 7 to 15 carbon atoms or halogen; and
R₁, R₂ and R₃ are each independently hydrogen; alkyl of 1 to 12 carbon atoms; hydroxy; alkoxy of 1 to 12 carbon atoms; halogen; phenyl or phenyl substituted by alkyl of 1 to 12 carbon atoms, hydroxy, alkoxy of 1 to 12 carbon atoms or halogen;
which process comprises
reacting the starting compound with a resorcinol of formula VI 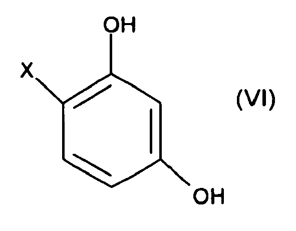 in the presence of an effective amount of a protic acid.

5. A process according to claim 1 wherein the leaving group E₁, E₂ and E₃ is Cl.

6. A process according to claim 3 or 5 in which no acid catalyst is added to the reaction mixture.

7. A process according to claim 4 in which the amount of resorcinol of formula VI used is
one to ten equivalents per equivalent of the compound of formula XIII for the preparation of a compound of the formula I or
two to twenty equivalents per equivalent of the compound of formula XIV for the preparation of a compound of the formula VII or
three to thirty equivalents per equivalent of the compound of formula B for the preparation of a compound of the formula XII.

8. A process according to claim 1 in which the protic acid is a hydrogen halide, sulfuric or a sulfonic acid.

9. A process according to claim 1 wherein the catalyst is 0.8 to 20 equivalents of the protic acid and wherein 0 to 0.25 equivalents of Lewis acid are added as a potentiator, each amount per equivalent of leaving group.

10. A process according to claim 4 wherein the reaction is carried out at a temperature of 70°C to 200°C.

11. A process for preparing a compound of formula A 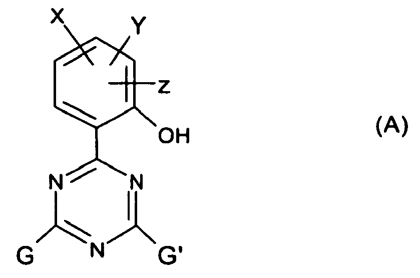 wherein
G is a residue of the formula 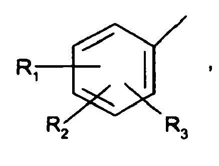 and
G' is a residue of the formula 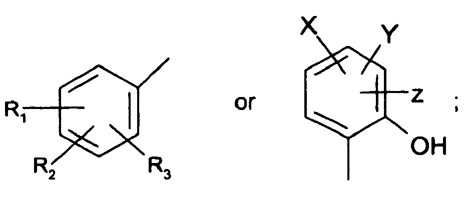
R₁, R₂ and R₃ are each independently hydrogen; alkyl of 1 to 12 carbon atoms; hydroxy; alkoxy of 1 to 12 carbon atoms; C₂-C₈alkenyl; C₃-C₁₈alkoxy interrupted by O and/or substituted by OH; C₂-C₁₈alkyl or C₂-C₁₈alkoxy, each interrupted by COO or substituted by COOH; halogen; nitro; amino; amino substituted by a C₁-C₁₂ hydrocarbon selected from alkyl, phenyl, cycloalkyl; C₂-C₁₈acylamino; C₂-C₁₂alkoxycarbonyl; C₅-C₁₂cycloalkyloxycarbonyl; C₇-C₁₅phenylalkoxycarbonyl; phenyl or phenyl substituted by alkyl of 1 to 12 carbon atoms, hydroxy, alkoxy of 1 to 12 carbon atoms or halogen; and
X, Y and Z, independently, are as defined for R₁, R₂ or R₃, or are phenylalkyl of 7 to 15 carbon atoms;
which process comprises
(1.) reacting a compound of the formula 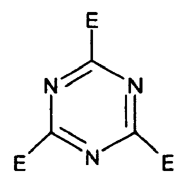 wherein
E is halogen, with a phenolic compound of formula II 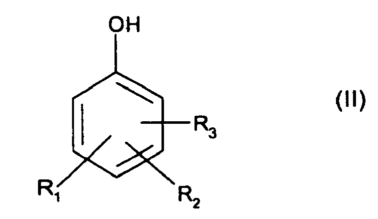
in the presence of a base to form a compound of formula III 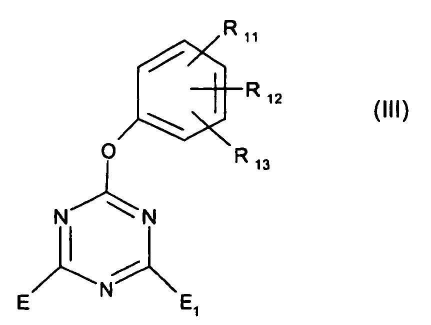 wherein R₁₁, R₁₂ and R₁₃, independently, are as defined for R₁, R₂ or R₃, and R₁₁ also may be
a residue of the formula 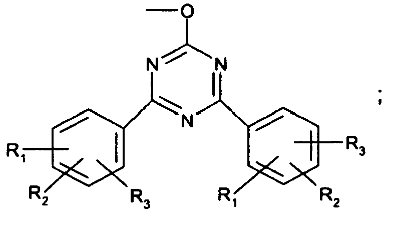 and
E₁ is halogen or a residue of the formula 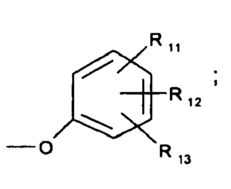
(2). reacting the compound of formula III neat with an excess of a compound of formula IV 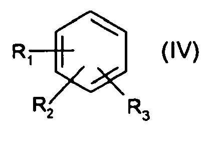 in the presence of an effective amount of a Lewis acid catalyst to give a compound of formula V 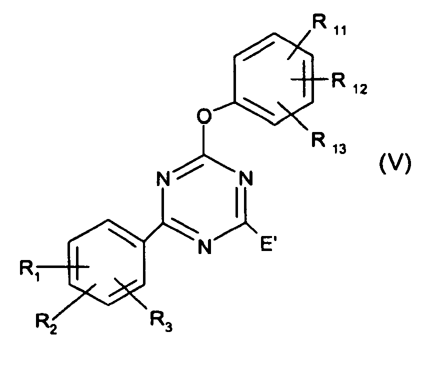 where E' is a residue of the formula 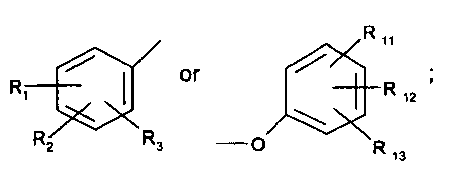
(3). reacting the compound of formula V neat with a compound of formula VI 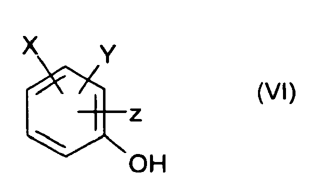 in the presence of an effective amount of a protic acid catalyst as the only catalyst to give the compound of formula A.

12. A process according to claim 11 wherein when G' in formula A is a residue of the formula 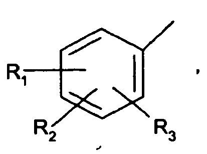 step 1 is run at -20°C to 100°C, and at least two equivalents of the compound of formula IV are used in step 2, and 1.2 to ten equivalents of the compound of formula VI are used per one equivalent of triazine in step 3, and when none of R₁, R₂ or R₃ in formula II is hydroxy, in step 1 one equivalent of the phenolic compound of formula II, and when one of R₁, R₂ or R₃ is hydroxy, in step 1 half an equivalent of the phenolic compound of formula II is used to form a compound of formula III 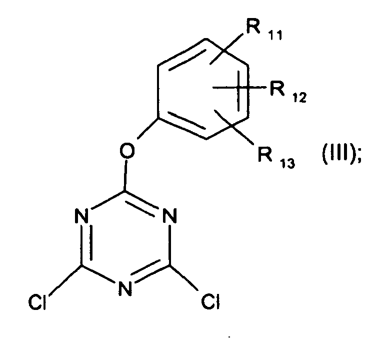 and when G' in formula A is a residue of the formula 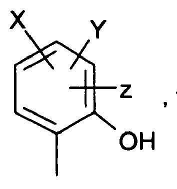 two equivalents of the phenolic compound of formula II used in step 1 to form a compound of formula VIII 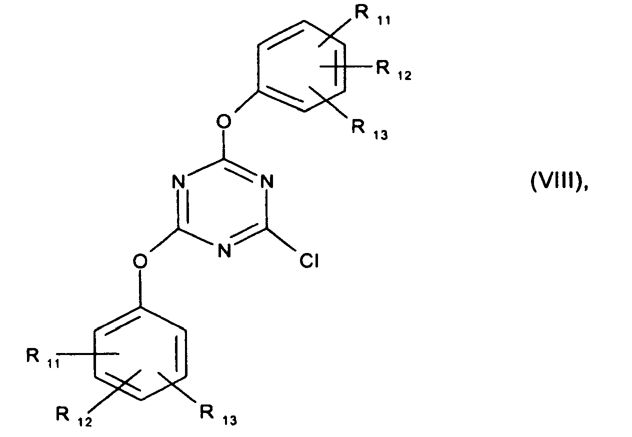 and step 1 run at -20°C to 200°C; 2.4 to twenty equivalents of the compound of formula VI are used per one equivalent of triazine in step 3.

13. A process according to claim 11 wherein the phenolic compound of formula II and the compound of formula VI are identical and are of the formula

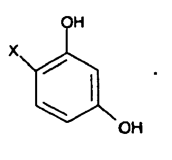

## Patentansprüche

1. Verfahren zur Herstellung einer Verbindung der Formel A 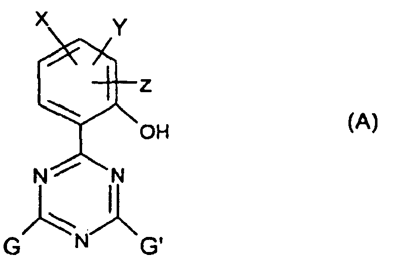 worin
G und G' jeweils unabhängig einen Rest der Formel 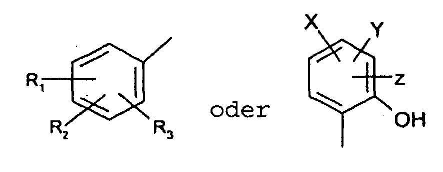 darstellen;
R₁, R₂ und R₃ jeweils unabhängig darstellen Wasserstoff; Alkyl mit 1 bis 12 Kohlenstoff-Atomen; Hydroxy; Alkoxy mit 1 bis 12 Kohlenstoff-Atomen; C₂-C₈-Alkenyl; C₃-C₁₈-Alkoxy, unterbrochen durch O und/oder substituiert mit OH; C₂-C₁₈-Alkyl oder C₂-C₁₈-Alkoxy, jeweils unterbrochen durch COO oder substituiert mit COOH; Halogen; Nitro; SO₃H; SO₃⁻; CN; Amino; Amino, substituiert mit einem C₁-C₁₂-Kohlenwasserstoff, ausgewählt aus Alkyl, Phenyl, Cycloalkyl; C₂-C₁₈-Acylamino; C₂-C₁₂-Alkoxycarbonyl; C₅-C₁₂-Cycloalkyloxy-carbonyl; C₇-C₁₅-Phenylalkoxycarbonyl; Phenyl oder Phenyl, substituiert mit Alkyl mit 1 bis 12 Kohlenstoff-Atomen, Hydroxy, Alkoxy mit 1 bis 12 Kohlenstoff-Atomen oder Halogen; oder R₁ und R₂ vicinal stehen und zusammen mit den Kohlenstoff-Atomen, an die sie gebunden sind, einen carbocyclischen Ring mit 5 oder 6 Kohlenstoff-Atomen, oder einen Ring mit 4 oder 5 Kohlenstoff-Atomen und -O-, -N= oder -NH- bilden; und
X, Y und Z unabhängig wie für R₁, R₂ oder R₃ definiert sind, oder Phenylalkyl mit 7 bis 15 Kohlenstoff-Atomen darstellen; wobei das Verfahren umfasst
Umsetzen einer Verbindung der Formel B 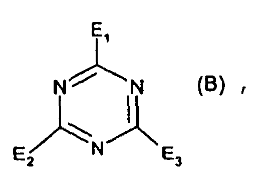
worin E₁ eine Abgangs-Gruppe darstellt, ausgewählt aus Halogen, C₁-C₁₂-Alkoxy oder 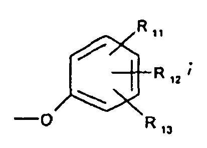 worin R₁₁, R₁₂ und R₁₃ wie für R₁, R₂ und R₃ definiert sind, und R₁₁ auch einen Rest der Formel 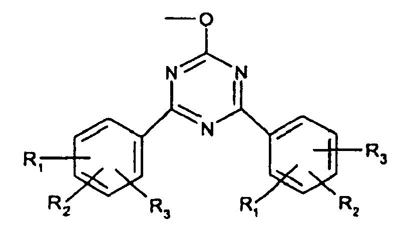 darstellen kann,
E₂ und E₃ eine Abgangs-Gruppe, wie für E₁ definiert, darstellen oder G oder G' darstellen;
mit einer Verbindung der Formel C 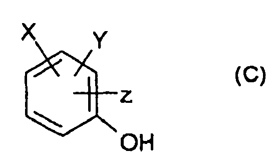 in Gegenwart einer wirksamen Menge von einem protischen SäureKatalysator, um die Verbindung der Formel A zu ergeben, wobei die Reaktion in der Schmelze ausgeführt wird.

2. Verfahren nach Anspruch 1, wobei in den Verbindungen der Formeln A, B und C
R₁, R₂ und R₃ jeweils unabhängig darstellen Wasserstoff; Alkyl mit 1 bis 12 Kohlenstoff-Atomen; Alkoxy mit 1 bis 12 Kohlenstoff-Atomen; C₂-C₈-Alkenyl; C₃-C₁₈-Alkoxy, unterbrochen durch O und/oder substituiert mit OH; C₂-C₁₈-Alkyl oder C₂-C₁₈-Alkoxy, jeweils unterbrochen durch COO oder substituiert mit COOH; Halogen; Nitro; Amino; Amino, substituiert mit einem C₁-C₁₂-Kohlenwasserstoff, ausgewählt aus Alkyl, Phenyl, Cycloalkyl; C₂-C₁₈-Acylamino; C₂-C₁₂-Alkoxycarbonyl; C₅-C₁₂-Cycloalkyloxy-carbonyl; C₇-C₁₅-Phenylalkoxycarbonyl; Phenyl oder Phenyl, substituiert mit Alkyl mit 1 bis 12 Kohlenstoff-Atomen, Hydroxy, Alkoxy mit 1 bis 12 Kohlenstoff-Atomen oder Halogen;
R₁₂ und R₁₃ wie für X und Y definiert sind, und
R₁₁ wie für Z definiert ist oder R₁₁ auch einen Rest der Formel 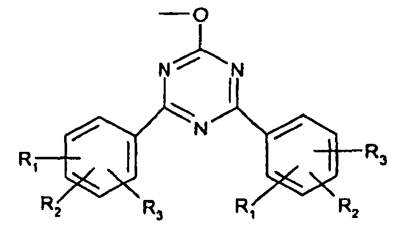 darstellen kann;
X und Y unabhängig Wasserstoff; Alkyl mit 1 bis 12 Kohlenstoff-Atomen; Phenylalkyl mit 7 bis 15 Kohlenstoff-Atomen darstellen; und
Z in meta-Position zu OH vorliegt und darstellt Hydroxy; Alkoxy mit 1 bis 12 Kohlenstoff-Atomen; C₃-C₁₈-Alkoxy, unterbrochen durch O und/oder substituiert mit OH; C₂-C₁₈-Alkoxy, unterbrochen durch COO oder substituiert mit COOH; Halogen; Nitro; Amino; Amino, substituiert mit einem C₁-C₁₂-Kohlenwasserstoff, ausgewählt aus Alkyl, Phenyl, Cycloalkyl; C₂-C₁₈-Acylamino; C₂-C₁₂-Alkoxycarbonyl.

3. Verfahren nach Anspruch 2, wobei in den Verbindungen der Formeln A, B und C
X Wasserstoff; Alkyl mit 1 bis 12 Kohlenstoff-Atomen; Phenylalkyl mit 7 bis 15 Kohlenstoff-Atomen oder Halogen darstellt;
Y Wasserstoff darstellt;
Z Hydroxy darstellt;
R₁, R₂ und R₃ jeweils unabhängig darstellen Wasserstoff; Alkyl mit 1 bis 12 Kohlenstoff-Atomen; Hydroxy; Alkoxy mit 1 bis 12 Kohlenstoff-Atomen; Halogen; Phenyl oder Phenyl, substituiert mit Alkyl mit 1 bis 12 Kohlenstoff-Atomen, Hydroxy, Alkoxy mit 1 bis 12 Kohlenstoff-Atomen oder Halogen;
E₁, E₂ und/oder E₃ als eine Abgangs-Gruppe in der Formel B Cl darstellen.

4. Verfahren nach Anspruch 1 zur Herstellung einer Verbindung der Formel I 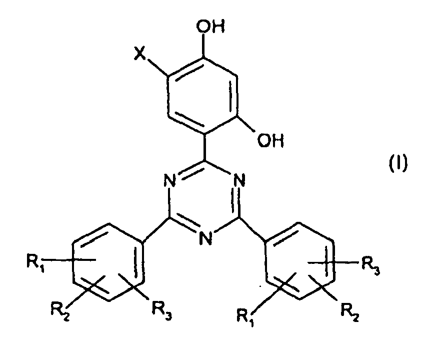 aus einer Ausgangs-Verbindung der Formel XIII 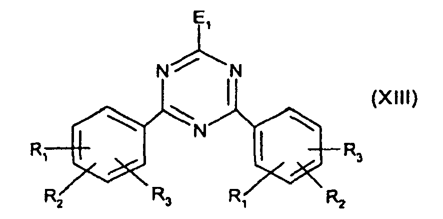 oder einer Verbindung der Formel VII 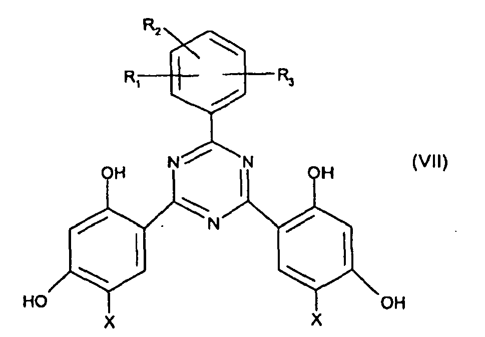 aus der Ausgangs-Verbindung der Formel XIV 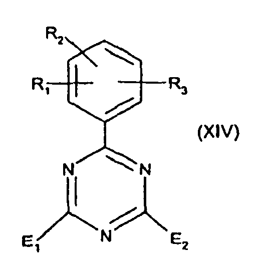 oder einer Verbindung der Formel XII 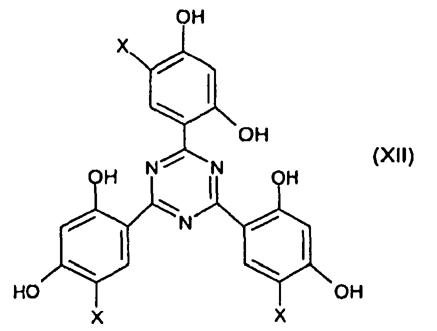 aus der Ausgangs-Verbindung der Formel B 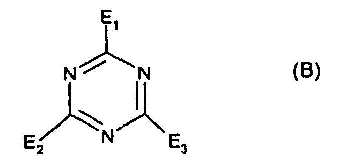 worin
E₁, E₂ und E₃ jeweils eine Abgangs-Gruppe darstellt, ausgewählt aus F, Cl und 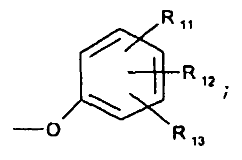 worin R₁₁, R₁₂ und R₁₃ wie für R₁, R₂ und R₃ definiert sind, und R₁₁, auch einen Rest der Formel 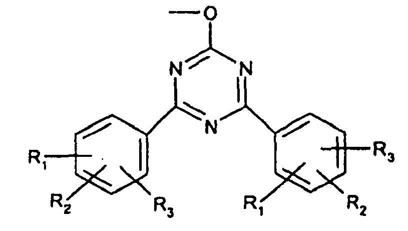 darstellen kann;
X Wasserstoff; Alkyl mit 1 bis 12 Kohlenstoff-Atomen; Phenylalkyl mit 7 bis 15 Kohlenstoff-Atomen oder Halogen darstellt; und R₁, R₂ und R₃ jeweils unabhängig darstellen Wasserstoff; Alkyl mit 1 bis 12 Kohlenstoff-Atomen; Hydroxy; Alkoxy mit 1 bis 12 Kohlenstoff-Atomen; Halogen; Phenyl oder Phenyl, substituiert mit Alkyl mit 1 bis 12 Kohlenstoff-Atomen, Hydroxy, Alkoxy mit 1 bis 12 Kohlenstoff-Atomen oder Halogen;
wobei das Verfahren umfasst
Umsetzen der Ausgangs-Verbindung mit einem Resorcin der Formel VI 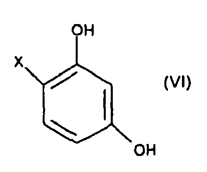 in Gegenwart einer wirksamen Menge von einer protischen Säure.

5. Verfahren nach Anspruch 1, wobei die Abgangs-Gruppe E₁, E₂ und E₃ Cl darstellt.

6. Verfahren nach Anspruch 3 oder 5, wobei kein Säure-Katalysator zu dem Reaktions-Gemisch gegeben wird.

7. Verfahren nach Anspruch 4, wobei die Menge an verwendetem Resorcin der Formel VI
ein bis zehn Äquivalente pro Äquivalent der Verbindung der Formel XIII für die Herstellung von einer Verbindung der Formel I oder
zwei bis zwanzig Äquivalente pro Äquivalent der Verbindung der Formel XIV für die Herstellung von einer Verbindung der Formel VII oder
drei bis dreißig Äquivalente pro Äquivalent der Verbindung der Formel B für die Herstellung von einer Verbindung der Formel XII ist.

8. Verfahren nach Anspruch 1, wobei die protische Säure eine Halogenwasserstoff-, Schwefel- oder eine Sulfonsäure ist.

9. Verfahren nach Anspruch 1, wobei der Katalysator 0,8 bis 20 Äquivalente von der protische Säure ist und wobei 0 bis 0,25 Äquivalente an Lewis-Säure als ein Potenziator, jede Menge pro Äquivalent Abgangs-Gruppe, zugegeben werden.

10. Verfahren nach Anspruch 4, wobei die Reaktion bei einer Temperatur von 70°C bis 200°C ausgeführt wird.

11. Verfahren zur Herstellung einer Verbindung der Formel A 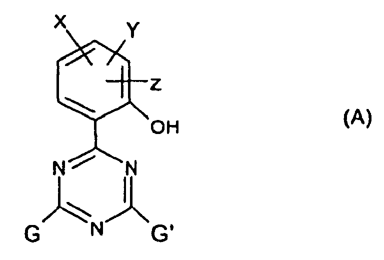 worin
G einen Rest der Formel 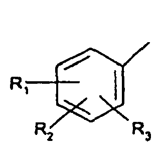 darstellt, und
G' einen Rest der Formel 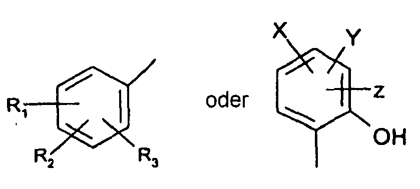 darstellt,
R₁, R₂ und R₃ jeweils unabhängig darstellen Wasserstoff; Alkyl mit 1 bis 12 Kohlenstoff-Atomen; Hydroxy; Alkoxy mit 1 bis 12 Kohlenstof f-Atomen; C₂-C₈-Alkenyl; C₃-C₁₈-Alkoxy, unterbrochen durch O und/oder substituiert mit OH; C₂-C₁₈-Alkyl oder C₂-C₁₈-Alkoxy, jeweils unterbrochen durch COO oder substituiert mit COOH; Halogen; Nitro; Amino; Amino, substituiert mit einem C₁-C₁₂-Kohlenwasserstoff, ausgewählt aus Alkyl, Phenyl, Cycloalkyl; C₂-C₁₈-Acylamino; C₂-C₁₂-Alkoxycarbonyl; C₅-C₁₂-Cycloalkyloxycarbonyl; C₇-C₁₅-Phenylalkoxycarbonyl; Phenyl oder Phenyl, substituiert mit Alkyl mit 1 bis 12 Kohlenstoff-Atomen, Hydroxy, Alkoxy mit 1 bis 12 Kohlenstoff-Atomen oder Halogen; und
X, Y und Z unabhängig wie für R₁, R₂ oder R₃ definiert sind, oder Phenylalkyl mit 7 bis 15 Kohlenstoff-Atomen darstellen;
wobei das Verfahren umfasst
(1.) Umsetzen einer Verbindung der Formel 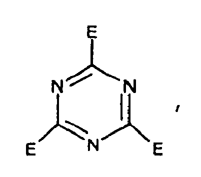 worin
E Halogen darstellt, mit einer phenolischen Verbindung der Formel II 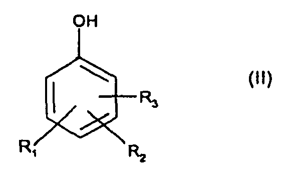 in Gegenwart von einer Base, um eine Verbindung der Formel III zu bilden 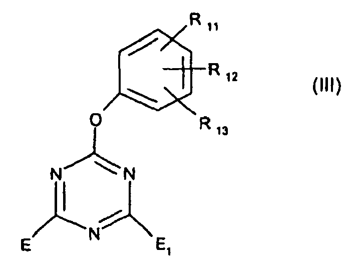 worin R₁₁, R₁₂ und R₁₃ unabhängig wie für R₁, R₂ oder R₃ definiert sind, und R₁₁ auch einen Rest der Formel 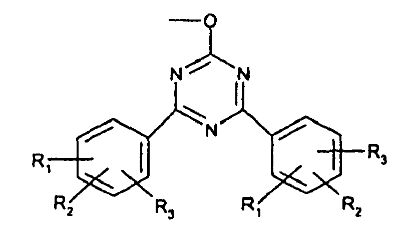 darstellen kann; und
E₁ Halogen oder einen Rest der Formel 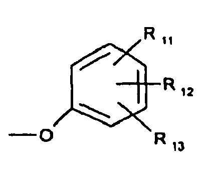 darstellt;
(2). Umsetzen der Verbindung der Formel III unverdünnt mit einem Überschuss von einer Verbindung der Formel IV 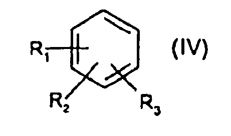 in Gegenwart einer wirksamen Menge von einem Lewis-Säure-Katalysator, um eine Verbindung der Formel V zu ergeben, 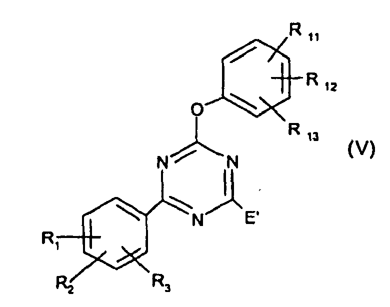 worin E' einen Rest der Formel 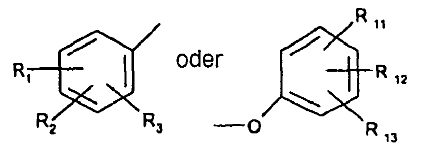 darstellt;
(3). Umsetzen der Verbindung der Formel V unverdünnt mit einer Verbindung der Formel VI 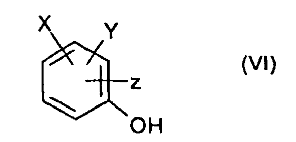 in Gegenwart einer wirksamen Menge von einem protischen SäureKatalysator als dem einzigen Katalysator, um eine Verbindung der Formel A zu ergeben.

12. Verfahren nach Anspruch 11, wobei wenn G' in Formel A einen Rest der Formel 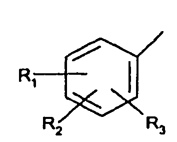 darstellt, Schritt 1 bei -20°C bis 100°C ausgeführt wird, und mindestens zwei Äquivalente der Verbindung der Formel IV in Schritt 2 verwendet werden, und 1,2 bis zehn Äquivalente der Verbindung der Formel VI pro ein Äquivalent von Triazin in Schritt 3 verwendet werden, und wenn keiner von R₁, R₂ oder R₃ in Formel II Hydroxy darstellt, in Schritt 1 ein Äquivalent der phenolischen Verbindung der Formel II, und wenn einer von R₁, R₂ oder R₃ Hydroxy darstellt, in Schritt 1 die Hälfte von einem Äquivalent der phenolischen Verbindung der Formel II verwendet wird, um eine Verbindung der Formel III 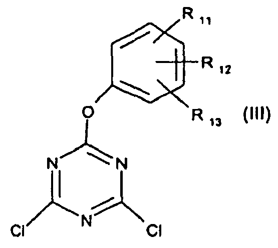 zu bilden; und wenn G' in Formel A einen Rest der Formel 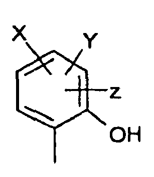 darstellt, zwei Äquivalente von der phenolischen Verbindung der Formel II in Schritt 1 verwendet werden, um eine Verbindung der Formel VIII 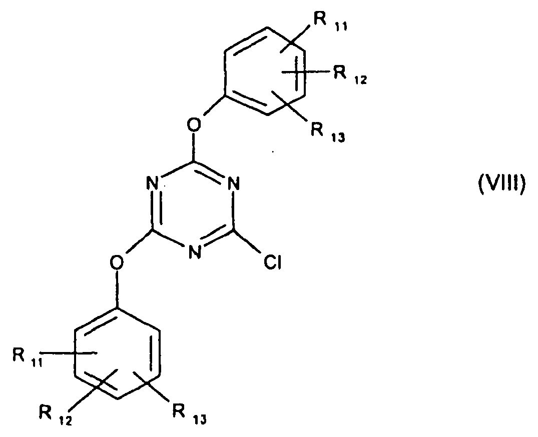 zu bilden;
und Schritt 1 bei -20°C bis 200°C abläuft; 2,4 bis zwanzig Äquivalente der Verbindung der Formel VI pro ein Äquivalent Triazin in Schritt 3 verwendet werden.

13. Verfahren nach Anspruch 11, wobei die phenolische Verbindung der Formel II und die Verbindung der Formel VI identisch sind und die Formel 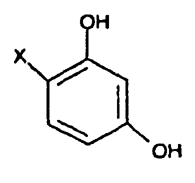 darstellen.

## Revendications

1. Procédé de préparation d'un composé de formule A 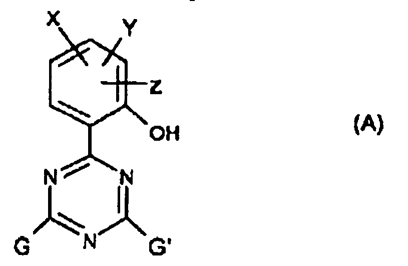 dans laquelle
G et G' sont chacun indépendamment un résidu de formule 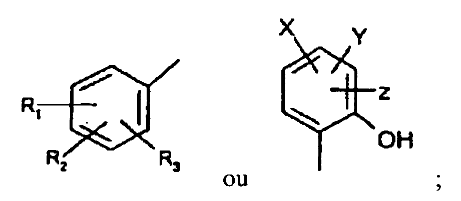
R₁, R₂ et R₃ sont chacun indépendamment un atome d'hydrogène ; un groupe alkyle de 1 à 12 atomes de carbone ; hydroxy ; alcoxy de 1 à 12 atomes de carbone ; alcényle en C₂ à C₈ ; alcoxy en C₃ à C₁₈ interrompu par O et/ou substitué par OH ; alkyle en C₂ à C₁₈ ou alcoxy en C₂ à C₁₈, chacun interrompu par COO ou substitué par COOH ; un atome d'halogène ; un groupe nitro ; SO₃H ; SO₃⁻ ; CN ; amino ; amino substitué par un hydrocarbure en C₁ à C₁₂ choisi parmi alkyle, phényle, cycloalkyle ; acylamino en C₂ à C₁₈ ; alcoxycarbonyle en C₂ à C₁₂ ; cycloalkyloxy en C₅ à C₁₂-carbonyle; phénylalcoxycarbonyle en C₇ à C₁₅ ; phényle ou phényle substitué par alkyle de 1 à 12 atomes de carbone, hydroxy, alcoxy de 1 à 12 atomes de carbone ou un atome d'halogène ; ou bien R₁ et R₂ sont viscinaux et forment, conjointement avec les atomes de carbone auxquels ils sont attachés, un cycle carbocyclique de 5 ou 6 atomes de carbone, ou un cycle de 4 ou 5 atomes de carbone et -O-, -N= ou -NH- ; et
X, Y et Z, indépendamment, sont tels que définis pour R₁, R₂ ou R₃, ou sont un groupe phénylalkyle de 7 à 15 atomes de carbone ;
lequel procédé comprend
la réaction d'un composé de formule B 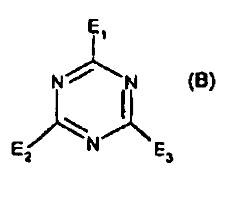 dans laquelle
E₁ est un groupe partant choisi parmi un atome d'halogène, un groupe alcoxy en C₁ à C₁₂ ou 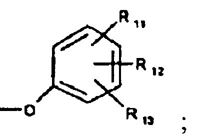
où R₁₁, R₁₂ et R₁₃ sont tels que définis pour R₁, R₂ et R₃, et R₁₁ peut également être un résidu de formule 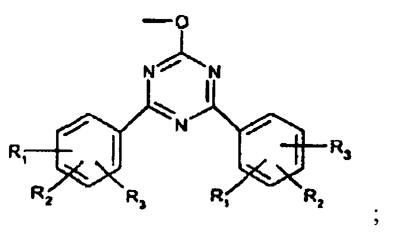
E₂ et E₃ sont un groupe partant tel que défini pour E₁ ou sont G ou G' ;
avec un composé de formule C 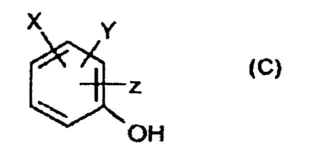 en présence d'une quantité efficace d'un catalyseur acide protique pour donner le composé de formule A, moyennant quoi la réaction est réalisée dans la matière fondue.

2. Procédé selon la revendication 1, dans lequel dans les composés de formules A, B et C
R₁, R₂ et R₃ sont chacun indépendamment un atome d'hydrogène ; un groupe alkyle de 1 à 12 atomes de carbone, alcoxy de 1 à 12 atomes de carbone ; alcényle en C₂ à C₈ ; alcoxy en C₃ à C₁₈ interrompu par O et/ou substitué par OH ; alkyle en C₂ à C₁₈ ou alcoxy en C₂ à C₁₈, chacun interrompu par COO ou substitué par COOH ; un atome d'halogène ; un groupe nitro ; amino ; amino substitué par un hydrocarbure en C₁ à C₁₂ choisi parmi alkyle, phényle, cycloalkyle ; acylamino en C₂ à C₁₈ ; alcoxycarbonyle en C₂ à C₁₂ ; cycloalkyloxy en C₅ à C₁₂-carbonyle ; phénylalcoxycarbonyle en C₇ à C₁₅ ; phényle ou phényle substitué par un alkyle de 1 à 12 atomes de carbone, par un hydroxy, par un alcoxy de 1 à 12 atomes de carbone ou par un atome d'halogène ;
R₁₂ et R₁₃ sont tels que définis pour X et Y ; et
R₁₁ est tel que défini pour Z ou R₁₁ peut également être un résidu de formule 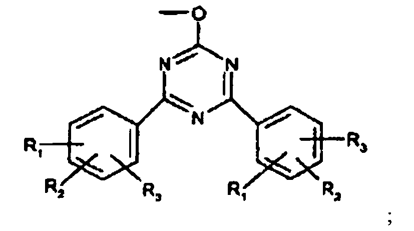 X et Y, indépendamment, sont un atome d'hydrogène ; un groupe alkyle de 1 à 12 atomes de carbone ; phénylalkyle de 7 à 15 atomes de carbone ; et
Z est en position méta par rapport à OH et est un groupe hydroxy ; alcoxy de 1 à 12 atomes de carbone ; alcoxy en C₃ à C₁₈ interrompu par O et/ou substitué par OH ; alcoxy en C₂ à C₁₈ interrompu par COO ou substitué par COOH ; un atome d'halogène ; un groupe nitro ; amino ; amino substitué par un hydrocarbure en C₁ à C₁₂ choisi parmi alkyle, phényle, cycloalkyle ; acylamino en C₂ à C₁₈ ; alcoxycarbonyle en C₂ à C₁₂.

3. Procédé selon la revendication 2, dans lequel, dans les composés de formules A, B et C
X est un atome d'hydrogène ; un groupe alkyle de 1 à 12 atomes de carbone ; phénylalkyle de 7 à 15 atomes de carbone ou un atome d'halogène ;
Y est un atome d'hydrogène ;
Z est un groupe hydroxy ;
R₁, R₂ et R₃ sont chacun indépendamment un atome d'hydrogène ; un groupe
alkyle de 1 à 12 atomes de carbone ; un groupe hydroxy ; un groupe alcoxy de 1 à 12 atomes de carbone; un atome d'halogène; un groupe phényle ou phényle substitué par un alkyle de 1 à 12 atomes de carbone, par un hydroxy, par un alcoxy de 1 à 12 atomes de carbone ou par un atome d'halogène ;
E₁, E₂ et/ou E₃ en tant que groupe partant dans la formule B sont Cl.

4. Procédé selon la revendication 1 pour préparer un composé de formule 1 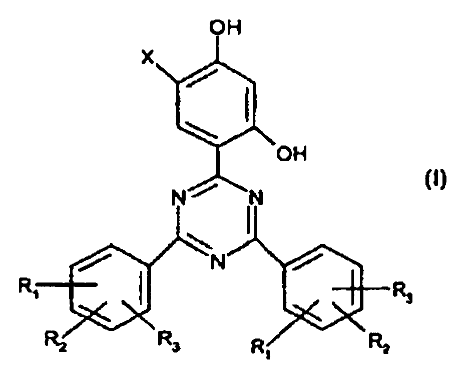 à partir d'un composé de départ de formule XIII 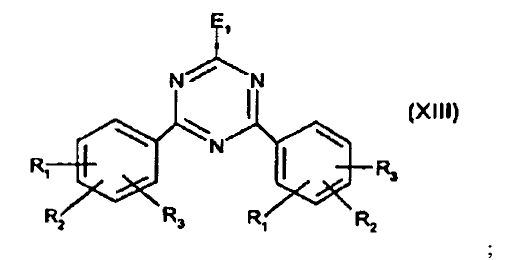 ou un composé de formule VII 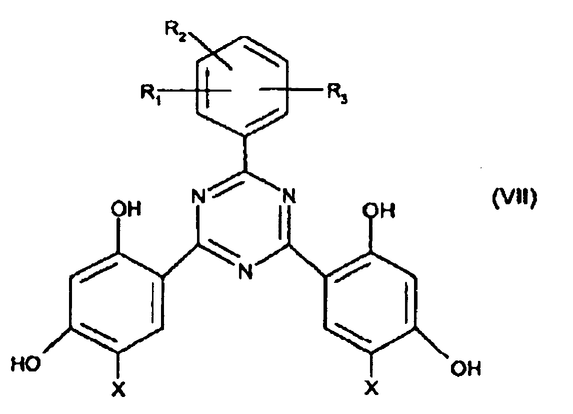 à partir du composé de départ de formule XIV 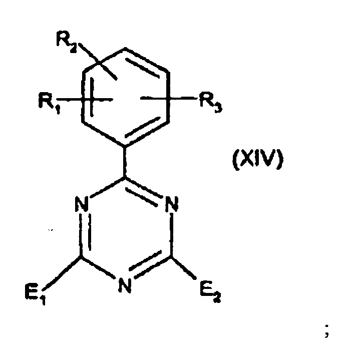 ou un composé de formule XII 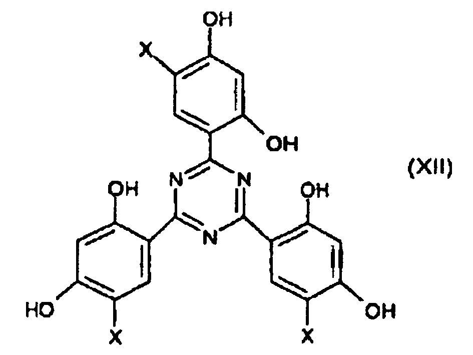 à partir du composé de départ de formule B 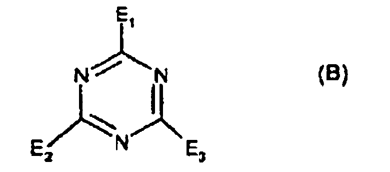 où
E₁, E₂ et E₃ sont chacun un groupe partant choisi parmi F, Cl et 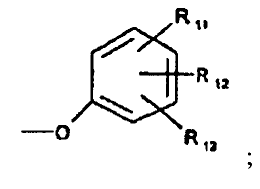
où R₁₁, R₁₂ et R₁₃ sont tels que définis pour R₁, R₂ et R₃, et R₁₁ peut également être un résidu de formule 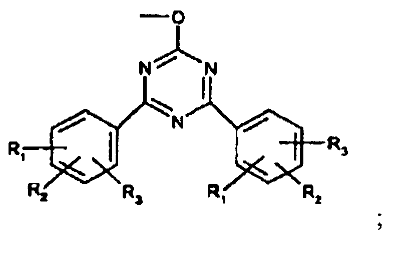
X est un atome d'hydrogène ; un groupe alkyle de 1 à 12 atomes de carbone ; phénylalkyle de 7 à 15 atomes de carbone ou un atome d'halogène ; et
R₁, R₂ et R₃ sont chacun indépendamment un atome d'hydrogène ; un groupe alkyle de 1 à 12 atomes de carbone ; hydroxy ; alcoxy de 1 à 12 atomes de carbone ; un atome d'halogène ; un groupe phényle, ou phényle substitué par un alkyle de 1 à 12 atomes de carbone, par un hydroxy, par un alcoxy de 1 à 12 atomes de carbone ou par un atome d'halogène ;
lequel procédé comprend
la réaction du composé de départ avec un résorcinol de formule VI 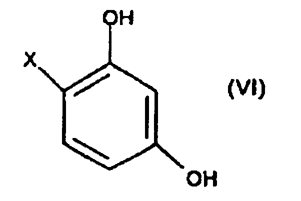 en présence d'une quantité efficace d'acide protique.

5. Procédé selon la revendication 1, dans lequel le groupe partant E₁, E₂ et E₃ est Cl.

6. Procédé selon la revendication 3 ou 5, dans lequel aucun catalyseur acide n'est ajouté au mélange de réaction.

7. Procédé selon la revendication 4, dans lequel la quantité de résorcinol de formule VI utilisée est de
un à dix équivalents par équivalent du composé de formule XIII pour la préparation d'un composé de formule I ou
deux à vingt équivalents par équivalent du composé de formule XIV pour la préparation d'un composé de formule VII ou
trois à trente équivalents par équivalent du composé de formule B pour la préparation d'un composé de formule XII.

8. Procédé selon la revendication 1, dans lequel l'acide protique est un halogénure d'hydrogène, l'acide sulfurique ou un acide sulfonique.

9. Procédé selon la revendication 1, dans lequel le catalyseur est de 0,8 à 20 équivalents de l'acide protique et dans lequel 0 à 0,25 équivalent d'acide de Lewis est ajouté en tant que renforçateur, chaque quantité étant par équivalent de groupe partant.

10. Procédé selon la revendication 4, dans lequel la réaction est réalisée à une température de 70 °C à 200 °C.

11. Procédé de préparation d'un composé de formule A 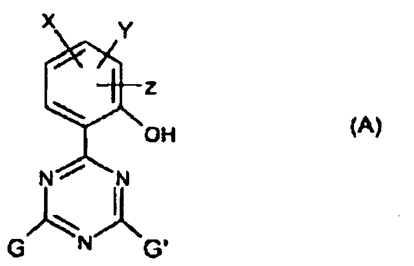 dans laquelle
G est un résidu de formule 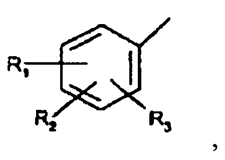 et
G' est un résidu de formule 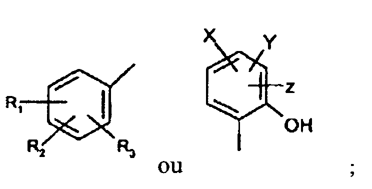
R₁, R₂ et R₃ sont chacun indépendamment un atome d'hydrogène ; un groupe alkyle de 1 à 12 atomes de carbone ; hydroxy ; alcoxy de 1 à 12 atomes de carbone ; alcényle en C₂ à C₈ ; alcoxy en C₃ à C₁₈ interrompu par O et/ou substitué par OH ; alkyle en C₂ à C₁₈ ou alcoxy en C₂ à C₁₈, chacun interrompu par COO ou substitué par COOH ; un atome d'halogène ; un groupe nitro ; amino ; amino substitué par un hydrocarbure en C₁ à C₁₂ choisi parmi alkyle, phényle, cycloalkyle ; acylamino en C₂ à C₁₈ ; alcoxycarbonyle en C₂ à C₁₂ ; cycloalkyloxy en C₅ à C₁₂-carbonyle ; phénylalcoxycarbonyle en C₇ à C₁₅ ; phényle ou phényle substitué par un alkyle de 1 à 12 atomes de carbone, par un hydroxy, par un alcoxy de 1 à 12 atomes de carbone ou par un atome d'halogène ; et
X, Y et Z, indépendamment, sont tels que définis pour R₁, R₂ ou R₃, ou sont un groupe phénylalkyle de 7 à 15 atomes de carbone ;
lequel procédé comprend
(1). la réaction d'un composé de formule 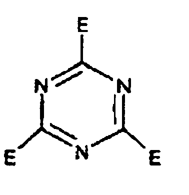 dans laquelle
E est un atome d'halogène, avec un composé phénolique de formule II 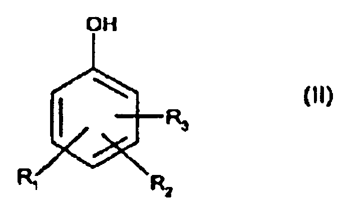 en présence d'une base pour former un composé de formule III 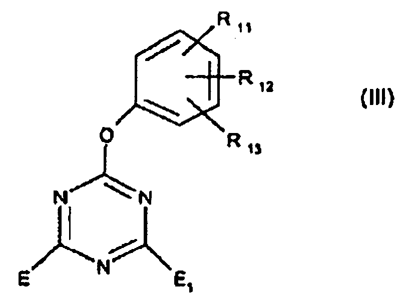 dans laquelle R₁₁, R₁₂ et R₁₃, indépendamment, sont tels que définis pour R₁, R₂ et R₃, et R₁₁ peut également être un résidu de formule 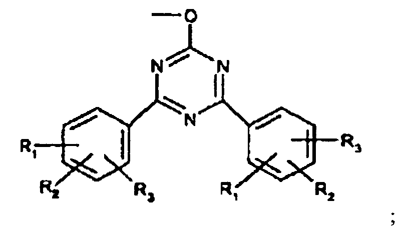 et
E₁ est un atome d'halogène ou un résidu de formule 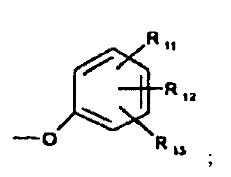
(2). la réaction du composé net de formule III avec un excès d'un composé de formule IV 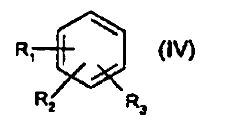 en présence d'une quantité efficace d'un catalyseur acide de Lewis pour donner un composé de formule V 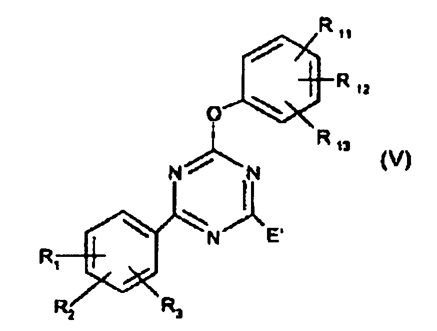 dans laquelle E' est un résidu de formule 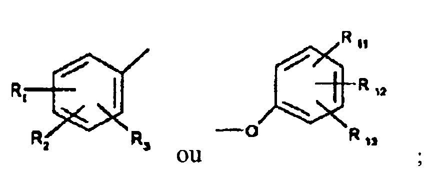
(3). la réaction du composé net de formule V avec un composé de formule VI 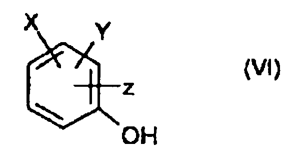 en présence d'une quantité efficace d'un catalyseur acide protique en tant que seul catalyseur pour donner le composé de formule A.

12. Procédé selon la revendication 11, dans lequel lorsque G' dans la formule A est un résidu de formule 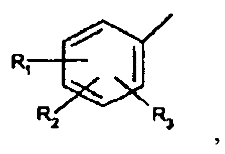 l'étape 1 est réalisée à une température de -20 °C à 100 °C, et au moins deux équivalents du composé de formule IV sont utilisés dans l'étape 2, et 1,2 à dix équivalents du composé de formule VI sont utilisés pour un équivalent de triazine dans l'étape 3, et lorsque aucun de R₁, R₂ ou R₃ dans la formule II n'est un groupe hydroxy, dans l'étape 1, un équivalent du composé phénolique de formule II est utilisé, et lorsque l'un de R₁, R₂ ou R₃ est un groupe hydroxy, dans l'étape 1, un demi-équivalent du composé phénolique de formule II est utilisé pour former un composé de formule III 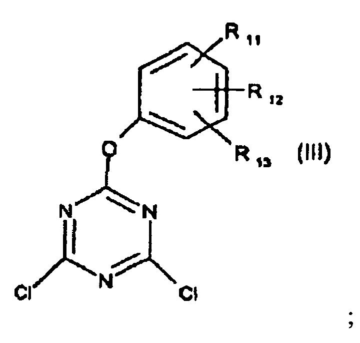 et lorsque G' dans la formule A est un résidu de formule 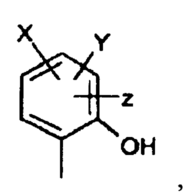 deux équivalents du composé phénolique de formule II sont utilisés dans l'étape 1 pour former un composé de formule VIII 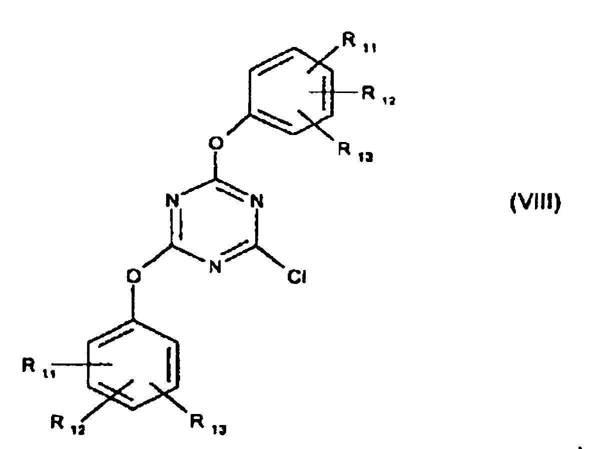 et l'étape 1 est réalisée à une température de -20 °C à 200 °C ; 2,4 à vingt équivalents du composé de formule VI sont utilisés pour un équivalent de triazine dans l'étape 3.

13. Procédé selon la revendication 11, dans lequel le composé phénolique de formule II et le composé de formule VI sont identiques et de formule
